# EUROPEAN PATENT SPECIFICATION

(11) **EP 4 222 170 B1**
(45) Date of publication and mention of the grant of the patent: **20.03.2024**
(21) Application number: 21786863.7
(22) Date of filing: 01.10.2021
(51) Int. Cl.: C07K 16/24

(54) **IMPROVED ANTI-OXMIF ANTIBODIES WITH REDUCED AGGREGATION POTENTIAL AND REDUCED HYDROPHOBICITY**
VERBESSERTE ANTI-OXMIF-ANTIKÖRPER MIT REDUZIERTEM AGGREGATIONSPOTENZIAL UND REDUZIERTER HYDROPHOBIZITÄT
ANTICORPS ANTI-OXMIF AMÉLIORÉS AVEC RÉDUCTION DU POTENTIEL D'AGRÉGATION ET DE L'HYDROPHOBICITÉ

(30) Priority: 02.10.2020 EP 20199829
(43) Date of publication of application: 09.08.2023
(73) Proprietor: OncoOne Research & Development GmbH, 3400 Klosterneuburg (AT)
(72) Inventor: SCHINAGL, Alexander, 1200 Vienna (AT); MIRKINA, Irina, 1220 Vienna (AT); KERSCHBAUMER, Randolf, 3400 Klosterneuburg (AT); THIELE, Robert Michael, 2201 Seyring (AT)
(74) Representative: Loidl, Manuela Bettina
(86) International application number: PCT/EP2021/077106
(87) International publication number: WO 2022/069712

(56) References cited:
- WO-A1-2009/086920
- WO-A1-2019/234241
- US-A1- 2011 236 375
- CHRISTIAN KELLNER ET AL: "Modulating Cytotoxic Effector Functions by Fc Engineering to Improve Cancer Therapy", TRANSFUSION MEDICINE HEMOTHERAPY, vol. 44, no. 5, 1 January 2017 (2017-01-01), pages 327-336, XP055612043, CH ISSN: 1660-3796, DOI: 10.1159/000479980

## Description

### FIELD OF THE INVENTION

The invention refers to anti-oxMIF antibodies with improved properties such as reduced aggregation potential and reduced hydrophobicity due to selected amino acid substitutions in the light and heavy chain variable domains and optionally increased effector functions due to further substitutions in the heavy chain constant regions, and their use in the treatment of oxMIF-related conditions.

### BACKGROUND OF THE INVENTION

The cytokine Macrophage Migration Inhibitory Factor (MIF) has been described as early as 1966 (David, J.R., 1966; Bloom B.R. and Bennet, B., 1966). MIF, however, is markedly different from other cytokines and chemokines because it is constitutively expressed, stored in the cytoplasm and present in the circulation of healthy subjects. Due to the ubiquitous nature of this protein, MIF can be considered as an inappropriate target for therapeutic intervention. However, MIF occurs in two immunologically distinct conformational isoforms, termed reduced MIF (redMIF) and oxidized MIF (oxMIF) (Thiele M. et al., 2015). RedMIF was found to be the abundantly expressed isoform of MIF that can be found in the cytoplasm and in the circulation of any subject. RedMIF seems to represent a latent non-active storage form (Schinagl. A. et al., 2018).

In contrast, oxMIF seems to be the physiologic relevant and disease related isoform which can be detected in tumor tissue, specifically in tumor tissue from patients with colorectal, pancreatic, ovarian and lung cancer outlining a high tumor specificity of oxMIF (Schinagl. A. et al., 2016), but also in the circulation of patients with inflammatory diseases (Thiele et al., 2015).

The number of successful drug targets to treat cancers, like the above mentioned oxMIF positive indications, is restricted. E.g. more than 300 potential immune-oncology targets are described, but many clinical studies focus on anti-PD1 and anti-PDL1 antibodies (Tang J., et al. 2018). The scientific and medical community therefore eagerly awaits potential drugs targeting tumor specific antigens to increase the therapeutic options for cancer patients with poor prognosis.

Monoclonal anti MIF antibodies are described in WO2009/086920A1.

Antibodies targeting oxMIF showed efficacy in *in vitro-* and *in vivo* models of inflammation and cancer (Hussain F. et al., 2013; Schinagl. A. et al., 2016; Thiele et al., 2015). An oxMIF specific antibody (Imalumab) demonstrated an acceptable safety profile, satisfactory tissue penetration and indications for anti-tumor activity in a phase 1 clinical trial (Mahalingam D. et al., 2015; Mahalingam D. et al. 2020).

Bispecific anti-oxMIF/anti CD3 antibodies are disclosed in WO2019/234241A1.

Protein aggregation, specifically antibody aggregation is frequently observed at several stages of bioprocessing, including protein expression, purification and storage. Antibody aggregation can affect the overall yield of therapeutic protein manufacturing processes and may contribute to stability and immunogenicity of therapeutic antibodies.

Protein aggregation of antibodies thus continues to be a significant problem in their developability and remains a major area of focus in antibody production. Antibody aggregation can be triggered by partial unfolding of its domains, leading to monomer-monomer association followed by nucleation and aggregate growth. Although the aggregation propensities of antibodies and antibody-based proteins can be affected by the external experimental conditions, they are strongly dependent on the intrinsic antibody properties as determined by their sequences and structures.

For example, resistance to aggregation can be achieved by stabilizing the native state (i.e., resisting unfolding) or by reducing the propensity of the unfolded or partially folded states of the protein to aggregate. A disadvantage of stabilizing the native state is that proteins will likely be exposed to an environment in which they will unfold. Generally, when a protein is denatured or unfolds, amino acid residues that normally mediate intramolecular contacts in the interior of the protein are exposed. Such exposure often makes proteins prone to form intermolecular contacts and aggregate. In contrast to proteins that resist unfolding, a protein having a reduced propensity to aggregate when unfolded will simply refold into a bioactive non-aggregated state after exposure to such an environment.

The aggregation-resistance or aggregation-propensity of antibodies and proteins comprising antigen binding domains thereof is usually limited by the most aggregation prone domain(s) contained therein and by the strength of its interaction with surrounding domains (if present).

This is because once that domain unfolds, if it is incapable of refolding, it may interact with other domains in the same protein or in other proteins and form aggregates. Constant domains of antibodies generally do not aggregate and do not vary considerably. Accordingly, the weakest domains of an antibody with regard to aggregation potential and stability are generally considered to be the variable domains (e.g., heavy chain variable domain (V_{H}) and/or light chain variable domain (V_{L}), Ewert S. et al., 2003). In this regard, incorporation of aggregation prone V_{H} or V_{L} domains into otherwise stable recombinant antibody products often imparts these generally undesirable traits to the new recombinant design. Thus, engineering a variable domain to be aggregation-resistant is most likely to render the entire protein which may comprise that variable domain aggregation-resistant. Various strategies have been proposed for reducing aggregation of variable domains, e.g., rational design of aggregation-resistant proteins, complementarity determining region (CDR) grafting, or introducing disulfide bonds into a variable domain. Rational design of aggregation-resistant proteins generally involves using in silico analysis to predict the effect of a point mutation on the aggregation propensity of a protein. However, there are several difficulties with this approach. For example, it is not sufficient to merely identify a mutation that is likely to reduce aggregation of an unfolded protein. Rather, the mutation must also not increase aggregation of a folded protein or affect the function of the folded protein, especially the binding properties such as affinity or specificity in case of antibodies. Furthermore, rational design requires detailed structural analysis of the specific protein being improved and thus, is difficult to use with a protein that has not been thoroughly characterized and is not readily applicable to a variety of different proteins. CDR grafting involves transplanting CDRs from one variable domain onto framework regions (FRs) of another variable domain. This strategy was shown to be useful in stabilizing an anti-EGP-2 scFv (Willuda J. et al., 1999). Disadvantages of this approach include the reduction in affinity that can occur following CDR grafting. This loss of affinity can be overcome by introducing mutations to the FRs, however such mutations can produce immunogenic epitopes in the protein, thereby making the protein undesirable from a therapeutic point of view. Furthermore, CDR grafting generally requires analysis of crystal structure or homology modeling of the donor and acceptor variable domains to assess suitability for grafting. Such an approach is laborious and requires specialized knowledge. Moreover, since each variable domain has a different structure, the method is not readily applied across a variety of molecules. As for methods involving introducing disulfide bonds into a variable domain, while the bond may assist in the protein correctly refolding, it also introduces rigidity into the variable domain. Such rigidity can reduce the affinity of an antibody for an antigen. Moreover, not all variable domains can support the introduction of the requisite cysteine residues for disulfide bond formation without loss of affinity or without introducing an immunogenic epitope. Furthermore, formation of disulfide bonds under high protein concentrations can lead to protein aggregation, thus negating any potential positive effect of the bond.

Reduction in aggregation propensity, however, has been shown to be accompanied by an increase in expression titer, showing that reducing protein aggregation is beneficial throughout the development process and can lead to a more efficient path to clinical studies. For therapeutic proteins, aggregates are a significant risk factor for deleterious immune responses in patients and can form via a variety of mechanisms. Controlling aggregation can improve protein stability, manufacturability, attrition rates, safety, formulation, titers, immunogenicity, and solubility (Wei Li et al., 2016; Van der Kant R. et al., 2017).

Further intrinsic properties of proteins such as hydrophobicity also play important roles in antibody solubility. Low solubility of these therapeutic proteins due to surface hydrophobicity has been shown to render formulation development more difficult and may lead to poor bio-distribution, undesirable pharmacokinetics behavior and immunogenicity *in vivo.* Decreasing the overall surface hydrophobicity of candidate monoclonal antibodies could thus provide benefits and cost savings relating to purification and dosing regimens.

It is believed that multiple mechanisms are involved in the anti-cancer effects. In particular, Fc gamma receptor (FcyR) IIIA-dependent and FcyRIIA-dependent effector functions may be two of the major critical mechanisms responsible for the clinical efficacy of therapeutic Abs. FcyRIIIA is known to be a major triggering receptor of antibody-dependent cell-mediated cytotoxicity (ADCC) in natural killer (NK) cells and FcyRIIA is known to be a major triggering receptor of antibody-dependent cellular phagocytosis (ADCP) in macrophages. Complement-dependent cytotoxicity (CDC), another effector function of antibody, is also considered a possible anti-tumor mechanism. Most therapeutic antibodies that have been licensed and developed as medical agents are of the human IgG1 isotype, which can induce ADCC and CDC. These effector functions are activated through the interactions of the Fc with either FcyRs or complements, and the interactions are affected by N-linked biantennary complex-type oligosaccharides attached to the antibody Fc region (Natsume et al., 2009). Several *in vitro* and *in vivo* studies have demonstrated that antibody-dependent cell-mediated cytotoxicity (ADCC) is the predominant mode of action against cancer cells. The requirement for a direct interaction between Ab and receptors for the Fc region of the antibodies (FcR) has been demonstrated for anti-tumor effects of these antibodies. Consequently, FcR-bearing immune effector cells play an important role in mediating their effects (lanello A. and Ahmad A., 2005). However, MIF antibodies known in the art neither induce complement-dependent cell lysis of MIF-responsive cells nor antibody-dependent cellular cytotoxicity (Hussain et al., 2013).

US2011/236375A1 describes Fc variants with enhanced effector function.

A combination of reduced aggregation potential and reduced hydrophobicity would render the antibodies' properties highly advantageous. There is a need in the art for aggregation-resistant antibodies against oxMIF with reduced hydrophobicity but also for anti-oxMIF antibodies with enhanced effector functions, which has not yet been met by current antibody modifications.

### SUMMARY OF THE INVENTION

It is the objective of the present invention to provide improved antibodies or antigen binding fragments thereof targeting oxMIF and having reduced aggregation propensity and hydrophobicity.

The objective is solved by the subject matter of the present invention.

The present invention discloses a recombinant anti-oxMIF antibody or an antigen binding fragment thereof having reduced aggregation potential and reduced hydrophobicity, comprising the following variable domains:
(a1) a light chain variable domain comprising SEQ ID NO:9 with 1, 2,3, 4, or 5 amino acid substitutions selected from M30L, F49Y, A51G, P80S, W93F, or
(a2) a light chain variable domain comprising SEQ ID NO:9 with 1, 2, 3, 4, or 5 amino acid substitutions selected from M30L, F49Y, A51G, P80S and W93F, and with 1, 2, 3, 4, or 5 further amino acid substitutions, with the proviso that the tyrosine at position 36 is preserved, and one of
(b1) a heavy chain variable domain comprising SEQ ID NO:6; or
(b2) a heavy chain variable domain comprising SEQ ID NO:6 with 1 or 2 amino acid substitutions selected from L5Q and W97Y, or
(b3) a heavy chain variable domain comprising SEQ ID NO:6 with 1 or 2 amino acid substitutions selected from L5Q and W97Y, and with 1, 2, 3, 4 or 5 further amino acid substitutions; wherein amino acid positions are numbered according to Kabat, and wherein the aggregation potential and hydrophobicity are reduced compared to an antibody or antigen binding fragment thereof comprising SEQ ID NO:6 and SEQ ID NO:9 lacking the amino acid substitutions.

Specifically, the recombinant anti-oxMIF antibody comprises the amino acid substitution W93F with reference to SEQ ID 9.

Specifically, the recombinant anti-oxMIF antibody comprises the amino acid substitution W97Y with reference to SEQ ID NO:6.

According to a further specific embodiment, the recombinant anti-oxMIF antibody comprises the amino acid substitutions W93F and W97Y.

Surprisingly, it had been shown that selected positions even within the CDR domains could be substituted to reduce aggregation potential and/or hydrophobicity without having any negative effect on the oxMIF binding affinity.

Specifically, the light chain variable domain comprises 1, 2, 3, 4 or all of the amino acid substitutions M30L, F49Y, A51G, P80S, W93F.

Specifically, the recombinant anti-oxMIF antibody comprises a constant light (CL) domain of SEQ ID NO: 38.

According to a specific embodiment, the recombinant anti-oxMIF antibody described herein also has enhanced effector functions and comprises a heavy chain constant region comprising
(a) SEQ ID NO:2 or a heavy chain constant region having at least 95% sequence identity to SEQ ID NO:2 and comprising amino acids 239D and 332E; or
(b) SEQ ID NO:3, or a heavy chain constant region having at least 95% sequence identity to SEQ ID NO:3 and comprising amino acids 239D, 268F, 324T and 332E; or
(c) SEQ ID NO:4, or a heavy chain constant region having at least 95% sequence identity to SEQ ID NO:4 and comprising amino acids 239D, and 332E; or
(d) SEQ ID NO:5, or a heavy chain constant region having at least 95% sequence identity to SEQ ID NO:5 and comprising amino acids 235V, 243L, 292P, 300L, and 396L, wherein amino acid positions are numbered according to EU numbering index

Specifically, the anti-oxMIF antibody described herein contains a VH or VL domain containing an amino acid sequence selected from the group consisting of SEQ ID NOs:7, 8, 10, 11, 12, and 13.

According to an alternative embodiment, the anti-oxMIF antibody contains SEQ ID NOs:6, 11 and any one of SEQ ID NOs:1, 2, 3, 4 or 5.

In a further embodiment, the anti-oxMIF antibody contains SEQ ID NOs:7, 10 and any one of SEQ ID NOs:1, 2, 3, 4 or 5.

In a further embodiment, the anti-oxMIF antibody contains SEQ ID NOs:7, 11 and any one of SEQ ID NOs:1, 2, 3, 4 or 5.

In a further embodiment, the anti-oxMIF antibody contains SEQ ID NOs:8, 10 and any one of SEQ ID NOs:1, 2, 3, 4, or 5.

Further described herein is an anti-oxMIF antibody which contains SEQ ID NOs:8, 11 and any one of SEQ ID NOs:1, 2, 3, 4, or 5, or SEQ ID No:39, or SEQ ID NO:40.

In a further embodiment, the anti-oxMIF antibody contains SEQ ID NOs:8, 13 and any one of SEQ ID NOs:1, 2, 3, 4, or 5, or SEQ ID No:41, or SEQ ID NO:42.

Further described herein is an anti-oxMIF antibody comprising SEQ ID NOs:39, 40, 41 or 42 or the anti-oxMIF antibody comprising SEQ ID NOs: 39, 44, 41 or 45

More specifically, the recombinant anti-oxMIF antibody or an antigen binding fragment thereof is a bispecific antibody.

In a specific embodiment, the anti-oxMIF antibody described herein is selected from the group consisting of monospecific and bispecific antibodies, scFv, (scFv)2, scFv-Fc, Fab, Fab', and F(ab')2, Fab'-SH, Fab-scFv fusion, Fab-(scFv)2-fusion, Fab-scFv-Fc, Fab-(scFv)2-Fc, fusion proteins of two single-chain variable fragments (scFvs) of different antibodies (e.g. BiTE), minibody, TandAb^{®}, DutaMab^{™}, DART, and CrossMab^{®}.

Specifically, the antibody as described herein is for use in the preparation of a medicament.

Specifically, a pharmaceutical composition is provided herein, comprising the antibody of the invention, optionally together with a pharmaceutical carrier or adjuvant.

Specifically, the composition comprises about 10 to 250 mg/ml of the antibody described herein, specifically comprising ≥ 50 mg/ml.

Specifically, said pharmaceutical composition is formulated for subcutaneous administration.

More specifically, the pharmaceutical composition can be used for the treatment of oxMlF-related conditions.

Specifically, the composition is for administration as the sole substance, or in combination with a further pharmaceutical composition comprising one or more active substances, preferably selected from the group consisting of antiviral, anti-inflammatory, anti-cancer, anti-angiogenic and antibiotic substances.

In a further embodiment, the pharmaceutical composition is for use in the treatment of a patient suffering from inflammatory disease, hyperproliferative disorder, infectious disease or cancer, specifically in the treatment of asthma, vasculitis, arthritis, sepsis, septic shock, endotoxic shock, toxic shock syndrome, acquired respiratory distress syndrome, glomerulonephritis, inflammatory bowel disease, Crohn's disease, ulcerative colitis, peritonitis, nephritis and psoriasis, colorectal cancer, ovarian cancer, breast cancer, prostate cancer, pancreas cancer, and lung cancer.

Further provided herein is an isolated nucleic acid encoding the antibody of the invention.

Also an expression vector is provided herein, comprising the nucleic acid molecule(s) described herein.

In a further embodiment, a host cell is provided, containing the nucleic acid or an expression vector described herein.

Further provided is a method of producing the antibody of the invention, comprising culturing a host cell and recovering said antibody from the cell culture.

Further provided herein is a method for producing an antibody of the invention, comprising expressing a nucleic acid encoding the antibody in a host cell.

### FIGURES

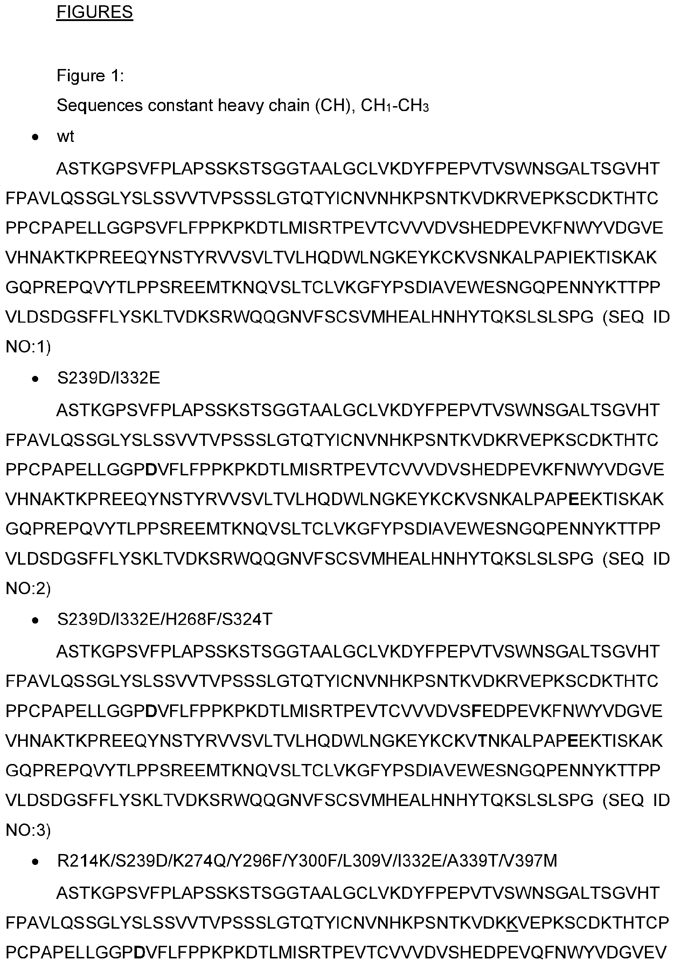

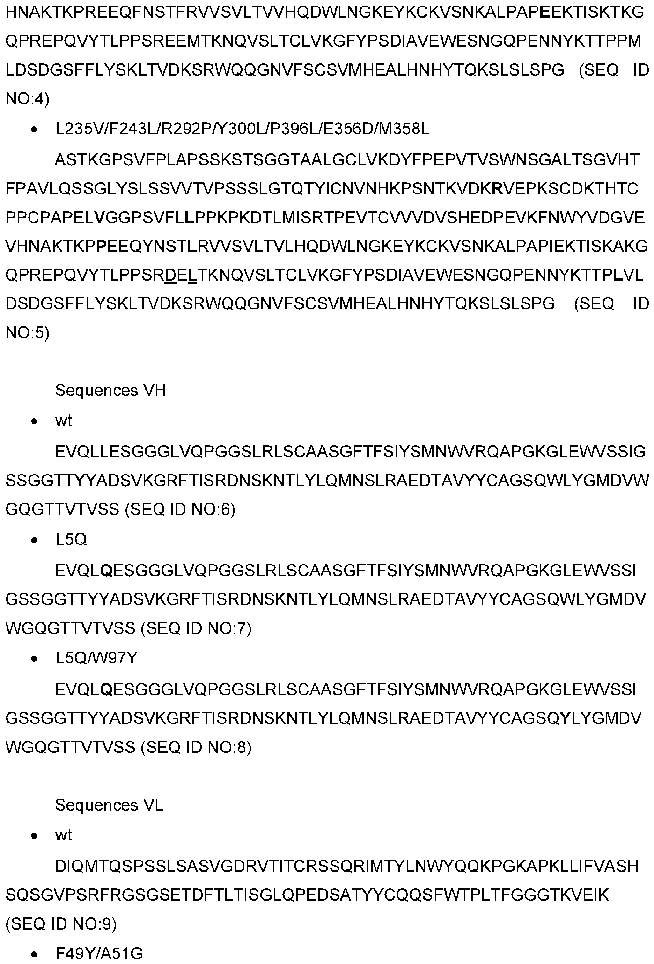

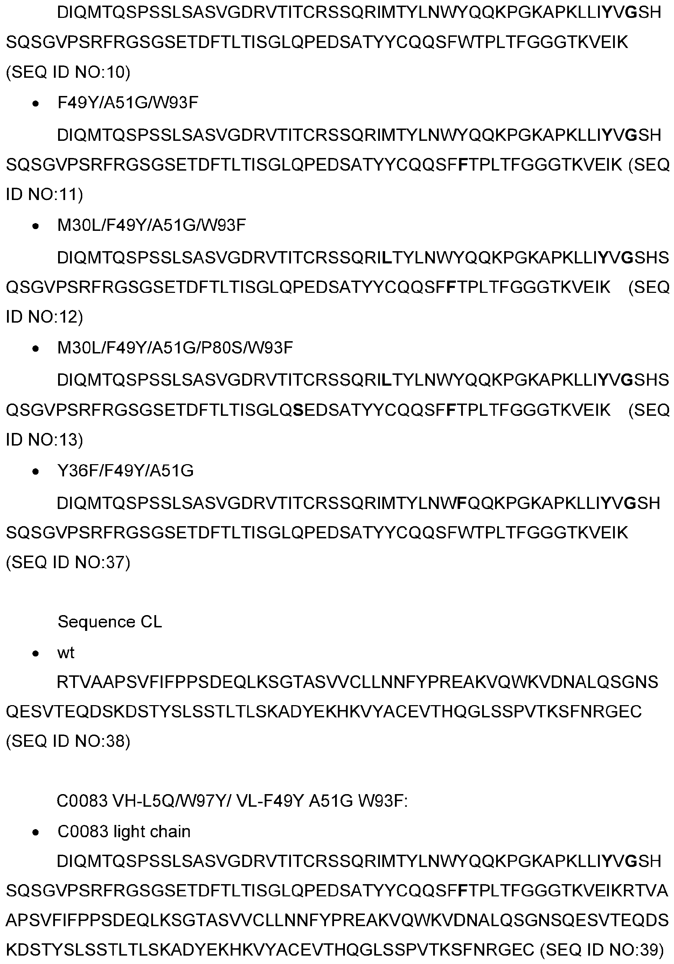

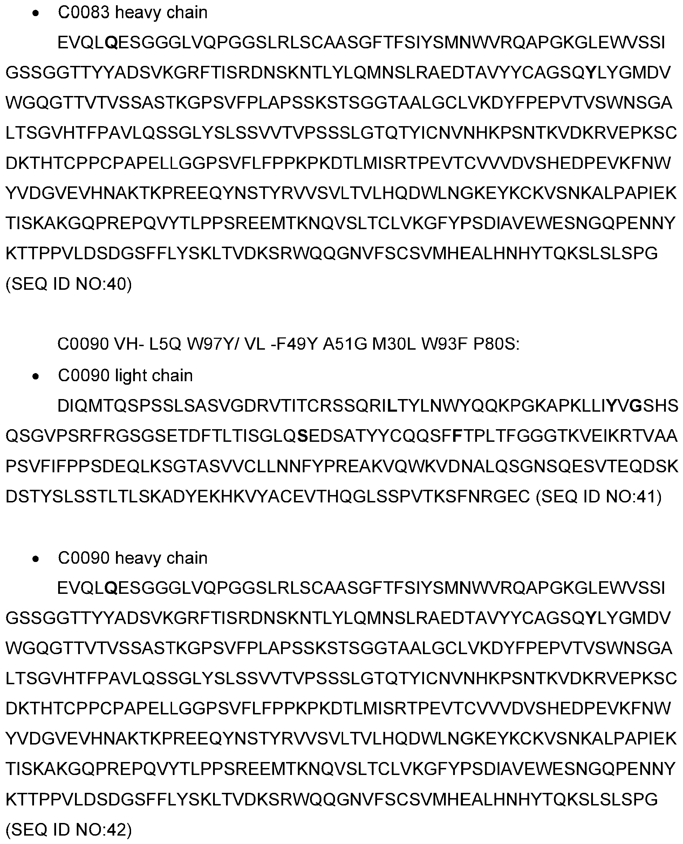

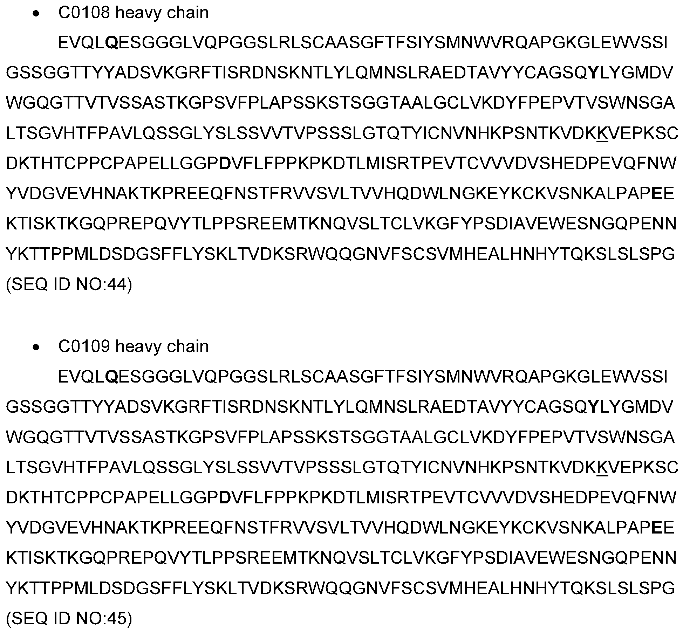

Figure 1: Amino acid sequences
Figure 2: Improved SEC (A, B) and HIC (C) profiles of newly designed anti-oxMIF antibodies.
Figure 3: Binding of newly designed anti-oxMIF antibodies to immobilized MIF (KD determination).
Figure 4: Differential binding of newly designed anti-oxMIF antibodies to oxMIF vs. redMIF.
Figure 5: Enhanced effector functions of newly designed anti-oxMIF antibodies in a modified ADCC Reporter Bioassay.
Figure 6: Tumor penetration of newly designed anti-oxMIF antibody C0083. A: Biodistribution of C0008 (5mg/kg/d); B: Biodistribution of C0083 (5mg/kg/d); C: non-treated control mice.
Figure 7: Improved production of newly designed anti-oxMIF antibody C0083.
Figure 8: Improved production of newly designed anti-oxMIF antibodies with enhanced effector functions and reduced hydrophobicity and aggregation propensity, compared to their respective counterparts with enhanced effector functions but comprising VH and VL domains of C0008 (comparison of C0097 vs C0064, C0098 vs C0065 and C0100 vs C0067).
Figure 9: Chromatography profiles demonstrating reduced aggregation and hydrophobicity of the newly designed anti-oxMIF antibodies. (A) Comparison of elution profiles of C0008 (control antibody, gray area) and newly designed antibodies C0108 and C0109 with enhanced effector functions on a Enrich 650 gel filtration column using 1xPBS as mobile phase; (B) Comparison of elution profiles of C0008 (control antibody, gray area) and newly designed antibodies C0083, C0090, C0108 and C0109 on a HiTrap Butyl HP HIC column.
Figure 10: Reduced unspecific binding of the newly designed anti-oxMIF antibodies on A2780 MIF^{-/-} cells determined by FACS. A2780 MIF^{-/-} cells were stained with the newly designed anti-oxMIF antibodies C0090, C0109 (A); C0083, C0108 (B) or the control antibody C0008 as well as an isotype IgG as negative control. GeoMean (mean fluorescence intensity for AF488), of viable cells was plotted against antibody concentration.
Figure 11: Binding curves of newly designed anti-oxMIF antibodies with enhanced effector functions to immobilized oxMIF (K_{D} estimation); anti-oxMIF antibody C0008 was used as reference antibody.
Figure 12: Differential binding of the newly designed antibodies to oxMIF vs. redMIF. C0008 was used as reference antibody and an isotype IgG as negative control.
Figure 13: Pharmacokinetic of the newly designed anti-oxMIF antibodies C0083 and C0090 in comparison to the control anti-oxMIF antibody C0008 in BALB/c nude mice. Plasma concentration of the antibodies was determined by a quantitative anti-oxMIF ELISA at 4, 10, 24, 48 and 96h post single intravenous injection of 10 mg/kg anti-oxMIF antibodies and the half-lives were calculated by a one-phase decay model in GraphPad Prism.

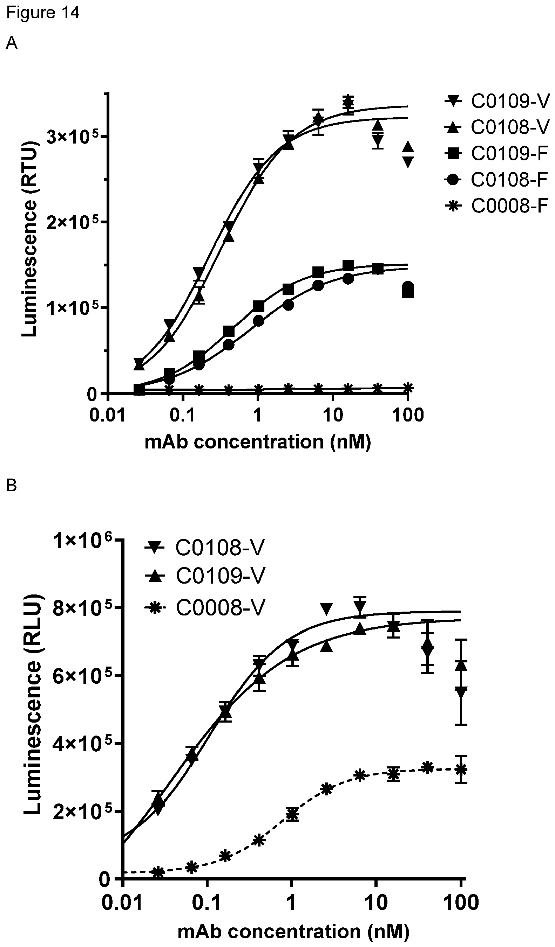

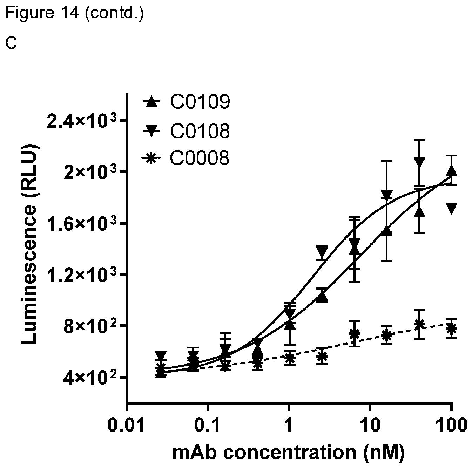

Figure 14: Enhanced effector functions of the newly designed antibodies C0108 and C0109 determined by reporter assays. (A-B) ADCC reporter bioassay with the newly designed antibodies C0108 and C0109 with enhanced effector functions using engineered Jurkat effector cells stably expressing FcyRIIIa (V high responder allotype, F low-responder allotype) and HCT116-pMIF (A) or A2780-pMIF (B) target cells compared to the anti-oxMIF control antibody C0008 with wt Fc; (C) ADCP reporter bioassay with the newly designed antibodies C0108 and C0109 with enhanced effector functions using engineered Jurkat effector cells stably expressing FcyRlla and HCT116-pMIF target cells compared to the anti-oxMIF control antibody C0008 with wt Fc. Mean and SEM are shown (n=2-4).
Figure 15: Enhanced ADCC activity of the newly designed anti-oxMIF antibodies C0108 and C0109 harboring ADCC/ADCP enhancing mutations in the Fc portion determined by a PBMC mediated cytotoxicity assay. ADCC bioassay with newly designed anti-oxMIF antibodies C0108 and C0109 and PBMCs as effector cells (A: 22% NK cells, B: 7% NK cells) and HCT116-pMIF as target cells compared to the anti-oxMIF control antibody C0008 with wt Fc. Mean and SEM of two or three replicates using PBMCs from two healthy donors are shown.
Figure 16: Newly designed anti-oxMIF antibodies show a strongly reduced unspecific cytokine release from human PBMCs. The newly designed anti-oxMIF antibodies C0108 and C0109 harboring ADCC/ADCP enhancing mutations in the Fc portion (70 nM) and the control anti-oxMIF antibody C0008 (70 nM) were incubated with human PBMCs overnight, and supernatants were analyzed in LegendPlex cytometric bead assays (BioLegend) for human IL-6 (A) and human MCP-1 (B). Mean +/- SEM for cytokine concentrations are shown from three different PMBC donors.

### DETAILED DESCRIPTION

The technical information set out below may in some respects go beyond the scope of the invention, which is defined exclusively by the appended claims. Such additional technical information which does not fall within the scope of the appended claims, is not part of the invention.

Unless indicated or defined otherwise, all terms used herein have their usual meaning in the art, which will be clear to the skilled person. Reference is for example made to the standard handbooks, such as Sambrook et al, "Molecular Cloning: A Laboratory Manual" (4th Ed.), Vols. 1 -3, Cold Spring Harbor Laboratory Press (2012); Krebs et al., "Lewin's Genes XI", Jones & Bartlett Learning, (2017), and Murphy & Weaver, "Janeway's Immunobiology" (9th Ed., or more recent editions), Taylor & Francis Inc, 2017.

The subject matter of the claims specifically refers to artificial products or methods employing or producing such artificial products, which may be variants of native (wild-type) products. Though there can be a certain degree of sequence identity to the native structure, it is well understood that the materials, methods and uses of the invention, e.g., specifically referring to isolated nucleic acid sequences, amino acid sequences, fusion constructs, expression constructs, transformed host cells and modified proteins, are "man-made" or synthetic, and are therefore not considered as a result of "laws of nature".

The terms "comprise", "contain", "have" and "include" as used herein can be used synonymously and shall be understood as an open definition, allowing further members or parts or elements. "Consisting" is considered as a closest definition without further elements of the consisting definition feature. Thus "comprising" is broader and contains the "consisting" definition.

The term "about" as used herein refers to the same value or a value differing by +/-5 % of the given value.

As used herein and in the claims, the singular form, for example "a", "an" and "the" includes the plural, unless the context clearly dictates otherwise.

As used herein, amino acids refer to twenty naturally occurring amino acids encoded by sixty-one triplet codons. These 20 amino acids can be split into those that have neutral charges, positive charges, and negative charges:
The "neutral" amino acids are shown below along with their respective three-letter and single-letter code and polarity: Alanine(Ala, A; nonpolar, neutral), Asparagine (Asn, N; polar, neutral), Cysteine (Cys, C; nonpolar, neutral), Glutamine (Gln, Q; polar, neutral), Glycine (Gly, G; nonpolar, neutral), Isoleucine (Ile, I; nonpolar, neutral), Leucine (Leu, L; nonpolar, neutral), Methionine (Met, M; nonpolar, neutral), Phenylalanine (Phe, F; nonpolar, neutral), Proline (Pro, P; nonpolar, neutral), Serine (Ser, S; polar, neutral), Threonine (Thr, T; polar, neutral), Tryptophan (Trp, W; nonpolar, neutral), Tyrosine (Tyr, Y; polar, neutral), Valine (Val, V; nonpolar, neutral), and Histidine (His, H; polar, positive (10%) neutral (90%)).

The "positively" charged amino acids are: Arginine (Arg, R; polar, positive), and Lysine (Lys, K; polar, positive).

The "negatively" charged amino acids are: Aspartic acid (Asp, D; polar, negative), and Glutamic acid (Glu, E; polar, negative).

The antibody or antigen-binding fragment of the invention comprises at least one binding site specifically recognizing oxMIF and exhibits reduced aggregation propensity and reduced hydrophobicity due to targeted amino acid substitutions in the variable heavy and light domains in comparison to the unmodified antibody lacking said amino acid substitutions as defined in the claims.

Reduction of aggregation potential is due to amino acid substitutions at selected positions within the variable domains of the antibody described herein.

The level of antibody aggregation can be measured using a variety of known techniques including mass spectrometry, size exclusion chromatography (SEC), hydrophobic interaction chromatography (HIC), dynamic light scattering (DLS), light obscuration (LO), dynamic imaging particle analysis (DIP A) techniques such as microflow imaging (MFI), and Coulter counter (CC), differential scanning fluorometry (DSF).

Reduced hydrophobicity and reduced aggregation potential as used herein refers to a reduction of the surface hydrophobicity and a reduced aggregation potential of the newly designed antibody compared to a reference antibody such as antibody C0008, which comprises SEQ ID Nos 1, 6, 38 and 9, as disclosed herein. The sequence of C0008 contains the sequence of Imalumab, published in the Proposed INN List 111 (WHO Drug Information, Vol. 28, No. 2, 2014), but lacking the C-terminal lysine. Measurement can be performed using a variety of known techniques including but not limited to hydrophobic interaction chromatography (HIC) or affinity-capture self-interaction nanoparticle spectroscopy (AC-SINS, Estep P. et al., 2015).

As described herein, the oxMIF antibody of the invention having reduced aggregation potential and reduced hydrophobicity specifically comprises a light chain variable domain having at least one of the amino acid substitutions M30L, F49Y, A51G, P80S, W93F according to the Kabat numbering , or a light chain variable domain with at least 95, specifically at least 96, 97, 98 or 99% sequence identity to SEQ ID NO:9 comprising the natural tyrosine at position 36 and furthermore at least one of the amino acid substitutions M30L, F49Y, A51G, P80S, W93F either in combination with a heavy chain variable domain comprising SEQ ID NO:6 or in combination with a heavy chain variable domain comprising SEQ ID NO:6 with the amino acid substitutions L5Q and/or W97Y according to the numbering of Kabat, or a heavy chain variable domain comprising SEQ ID NO:6 with 1, 2, 3, 4 or 5 amino acid substitutions and further comprising at least one of the amino acid substitutions L5Q or W97Y.

In an alternative embodiment, the light chain variable domain comprising SEQ ID NO:9 has 1, 2, 3, 4, 5 amino acid substitutions with the proviso that the natural tyrosine at position 36 is preserved and that furthermore at least one of the amino acids are substituted at positions M30, F49, A51, P80, W93.

Specifically, amino acid substitutions at positions W93 and W97 are preferred.

Further, an amino acid substitution at position F49 is preferred.

Further described herein is an anti-oxMIF antibody having reduced aggregation potential and reduced hydrophobicity, which specifically comprises a light chain variable domain comprising SEQ ID NO:9 or a light chain variable domain with at least 95% sequence identity to SEQ ID NO:9 comprising a tyrosine at position 36 and a heavy chain variable domain comprising SEQ ID NO:6 and amino acid substitution W97Y or amino acid substitutions L5Q and W97Y, or a heavy chain variable domain comprising SEQ ID NO:6 with 1, 2, 3, 4 or 5 amino acid substitutions and further comprising amino acid substitution W97Y, optionally in combination with L5Q.

The natural tyrosine at position 36 of the light chain is kept unmodified to preserve binding properties of the antibody described herein. Any modifications at said amino acid position may result in unwanted impaired binding properties.

Specifically, the variable light chain domain contains SEQ ID NO:10, and the variable heavy chain domain contains SEQ ID NO:7.

Specifically, the variable light chain domain contains SEQ ID NO:10, and the variable heavy chain domain contains SEQ ID NO:8.

Specifically, the variable light chain domain contains SEQ ID NO:11, and the variable heavy chain domain is of SEQ ID NO:6.

Specifically, the variable light chain domain contains SEQ ID NO:11, and the variable heavy chain domain contains SEQ ID NO:7.

Specifically, the variable light chain domain contains SEQ ID NO:11, and the variable heavy chain domain contains SEQ ID NO:8.

Specifically, the variable light chain domain contains SEQ ID NO:12, and the variable heavy chain domain is of SEQ ID NO:6.

Specifically, the variable light chain domain contains SEQ ID NO:12, and the variable heavy chain domain contains SEQ ID NO:8.

Specifically, the variable light chain domain contains SEQ ID NO:13, and the variable heavy chain domain is of SEQ ID NO:6.

Specifically, the variable light chain domain contains SEQ ID NO:13, and the variable heavy chain domain contains SEQ ID NO:8.

By "effector function" as used herein is meant a biochemical event that results from the interaction of an antibody Fc region with an Fc receptor or ligand. Effector functions include but are not limited to ADCC, ADCP, and CDC.

By "effector cell" as used herein is meant a cell of the immune system that expresses one or more Fc receptors and mediates one or more effector functions. Effector cells include but are not limited to monocytes, macrophages, neutrophils, dendritic cells, eosinophils, mast cells, platelets, B cells, large granular lymphocytes, Langerhans' cells, natural killer (NK) cells, and T cells, and may be from any organism including but not limited to humans, mice, rats, rabbits, and monkeys. In a specific embodiment, the anti-oxMIF antibodies described herein have also enhanced effector functions due to amino acid substitutions at selected positions in the heavy chain constant region. Increased effector functions of these antibodies due to enhanced complement- and FcyR-mediated activities can include enhanced complement dependent cytotoxicity (CDC), antibody-dependent cell-mediated cytotoxicity (ADCC) and/or antibody dependent cellular phagocytosis (ADCP). Fc receptor-mediated ADCC is an important mechanism of action by which antibodies target diseased cells for elimination. When the Fc effector portion of target-bound antibodies also binds to FcyRIIIA receptors on the cell surface of effector cells, multiple cross-linking of the two cell types occurs, leading to pathway activation of ADCC.

Antibodies with enhanced complement activities can be determined by cell-based CDC assays and improved binding to C1q determined by e.g. SPR or ELISA.

Improved or increased CDC activity is determined to be at least 1.5-fold, specifically at least 1.6-fold, 1.7-fold, 1.8-fold, 1.9-fold, 2-fold, 3-fold, 4-fold, more specifically at least 5-fold, more specifically at least 6-fold increase compared to a reference, i.e. unmodified, wild type antibody, e.g. C0008.

Increased ADCC activity is determined to be at least 1.5-fold, specifically at least 1.6-fold, 1.7-fold, 1.8-fold, 1.9-fold, 2-fold, 3-fold, 4-fold, more specifically at least 5-fold, more specifically at least 6-fold increased potency compared to a reference antibody, i.e. unmodified, wild type antibody, e.g. C0008.

In addition to the light and heavy variable domains described above, the oxMIF antibody may further comprise a heavy chain constant region comprising SEQ ID NO:1 with at least the amino acid substitutions S239D and I332E according to the EU numbering index, or a functional variant having at least 95%, specifically at least 96, 97, 98 or 99% sequence identity to SEQ ID NO:1 and comprising at least the amino acid substitutions S239D, I332E and optionally further amino acid substitutions H268F and/or S324T.

Specifically, the oxMIF-antibody contains a heavy chain constant region comprising SEQ ID NO:2 or a heavy chain constant region having at least 95% sequence identity to SEQ ID NO:2 and comprising amino acids 239D and 332E.

As an alternative, the oxMIF-antibody contains a heavy chain constant region comprising SEQ ID NO:3, or a heavy chain constant region having at least 95% sequence identity to SEQ ID NO:3 and comprising amino acids 239D, 268F, 324T and 332E.

Alternatively, the oxMIF antibody may further comprise a heavy chain constant region comprising SEQ ID NO:4, or a heavy chain constant region having at least 95% sequence identity to SEQ ID NO:4 and comprising amino acids 239D, and 332E.

In a further alternative embodiment, the anti-oxMIF antibody may further comprise a heavy chain constant region of SEQ ID NO:5, or a heavy chain constant region having at least 95% sequence identity to SEQ ID NO:5 and comprising amino acids 235V, 243L, 292P, 300L, and 396L.

In a further embodiment, the anti-oxMIF antibody comprises SEQ ID NOs:1, 7, 10, and 38. In a further embodiment, the anti-oxMIF antibody comprises SEQ ID NOs:2, 7, 10, and 38. In a further embodiment, the anti-oxMIF antibody comprises SEQ ID NOs:3, 7, 10, and 38. In a further embodiment, the anti-oxMIF antibody comprises SEQ ID NOs:4, 7, 10, and 38. In a further embodiment, the anti-oxMIF antibody comprises SEQ ID NOs:5, 7, 10, and 38. In a further embodiment, the anti-oxMIF antibody comprises SEQ ID NOs:1, 8, 10, and 38. In a further embodiment, the anti-oxMIF antibody comprises SEQ ID NOs:2, 8, 10, and 38. In a further embodiment, the anti-oxMIF antibody comprises SEQ ID NOs:3, 8, 10, and 38. In a further embodiment, the anti-oxMIF antibody comprises SEQ ID NOs:4, 8, 10, and 38. In a further embodiment, the anti-oxMIF antibody comprises SEQ ID NOs:5, 8, 10, and 38. In a further embodiment, the anti-oxMIF antibody comprises SEQ ID NOs:1, 6, 11, and 38. In a further embodiment, the anti-oxMIF antibody comprises SEQ ID NOs:2, 6, 11, and 38.

In a further embodiment, the anti-oxMIF antibody comprises SEQ ID NOs:3, 6, 11, and 38. In a further embodiment, the anti-oxMIF antibody comprises SEQ ID NOs:4, 6, 11, and 38. In a further embodiment, the anti-oxMIF antibody comprises SEQ ID NOs:5, 6, 11, and 38. In a further embodiment, the anti-oxMIF antibody comprises SEQ ID NOs:1, 7, 11, and 38. In a further embodiment, the anti-oxMIF antibody comprises SEQ ID NOs:2, 7, 11, and 38. In a further embodiment, the anti-oxMIF antibody comprises SEQ ID NOs:3, 7, 11, and 38. In a further embodiment, the anti-oxMIF antibody comprises SEQ ID NOs:4, 7, 11, and 38. In a further embodiment, the anti-oxMIF antibody comprises SEQ ID NOs:5, 7, 11, and 38. In a further embodiment, the anti-oxMIF antibody comprises SEQ ID NOs:1, 8, 11, and 38. In a further embodiment, the anti-oxMIF antibody comprises SEQ ID NOs:2, 8, 11, and 38. In a further embodiment, the anti-oxMIF antibody comprises SEQ ID NOs:3, 8, 11, and 38. In a further embodiment, the anti-oxMIF antibody comprises SEQ ID NOs:4, 8, 11, and 38. In a further embodiment, the anti-oxMIF antibody comprises SEQ ID NOs:5, 8, 11, and 38. In a further embodiment, the anti-oxMIF antibody comprises SEQ ID NOs:1, 6, 12, and 38. In a further embodiment, the anti-oxMIF antibody comprises SEQ ID NOs: 2, 6, 12 and 38. In a further embodiment, the anti-oxMIF antibody comprises SEQ ID NOs:3, 6, 12, and 38. In a further embodiment, the anti-oxMIF antibody comprises SEQ ID NOs:4, 6, 12, and 38. In a further embodiment, the anti-oxMIF antibody comprises SEQ ID NOs:5, 6, 12, and 38. In a further embodiment, the anti-oxMIF antibody comprises SEQ ID NOs:1, 7, 12, and 38. In a further embodiment, the anti-oxMIF antibody comprises SEQ ID NOs:2, 7, 12, and 38. In a further embodiment, the anti-oxMIF antibody comprises SEQ ID NOs:3, 7, 12, and 38. In a further embodiment, the anti-oxMIF antibody comprises SEQ ID NOs:4, 7, 12, and 38. In a further embodiment, the anti-oxMIF antibody comprises SEQ ID NOs:5, 7, 12, and 38. In a further embodiment, the anti-oxMIF antibody comprises SEQ ID NOs:1, 8, 12, and 38. In a further embodiment, the anti-oxMIF antibody comprises SEQ ID NOs:2, 8, 12, and 38. In a further embodiment, the anti-oxMIF antibody comprises SEQ ID NOs:3, 8, 12, and 38. In a further embodiment, the anti-oxMIF antibody comprises SEQ ID NOs:4, 8, 12, and 38. In a further embodiment, the anti-oxMIF antibody comprises SEQ ID NOs:5, 8, 12, and 38. In a further embodiment, the anti-oxMIF antibody comprises SEQ ID NOs:1, 6, 13, and 38. In a further embodiment, the anti-oxMIF antibody comprises SEQ ID NOs: 2, 6, 13 and 38. In a further embodiment, the anti-oxMIF antibody comprises SEQ ID NOs:3, 6, 13, and 38. In a further embodiment, the anti-oxMIF antibody comprises SEQ ID NOs:4, 6, 13, and 150. In a further embodiment, the anti-oxMIF antibody comprises SEQ ID NOs:5, 6, 13, and 38. In a further embodiment, the anti-oxMIF antibody comprises SEQ ID NOs:1, 7, 13, and 38. In a further embodiment, the anti-oxMIF antibody comprises SEQ ID NOs:2, 7, 13, and 38. In a further embodiment, the anti-oxMIF antibody comprises SEQ ID NOs:3, 7, 13, and 38. In a further embodiment, the anti-oxMIF antibody comprises SEQ ID NOs:4, 7, 13, and 38. In a further embodiment, the anti-oxMIF antibody comprises SEQ ID NOs:5, 7, 13, and 38. In a further embodiment, the anti-oxMIF antibody comprises SEQ ID NOs:1, 8, 13, and 38. In a further embodiment, the anti-oxMIF antibody comprises SEQ ID NOs:2, 8, 13, and 38. In a further embodiment, the anti-oxMIF antibody comprises SEQ ID NOs:3, 8, 13, and 38. In a further embodiment, the anti-oxMIF antibody comprises SEQ ID NOs:4, 8, 13, and 38. In a further embodiment, the anti-oxMIF antibody comprises SEQ ID NOs:5, 8, 13, and 38.

The oxMIF binding site of the antibody described herein is specific for the oxidized form of MIF, i.e. for animal, specifically for mammalian oxMIF, such as but not limited to mouse, rat, monkey and human, specifically for human oxMIF, but does not show substantial cross-reactivity to reduced MIF. oxMIF is the disease-related structural isoform of MIF which can be specifically and predominantly detected in the circulation of subjects with inflammatory diseases and in tumor tissue of cancer patients. In one embodiment, the humanized or human anti-oxMIF binding site comprises one or more (e.g., all three) light chain complementary determining regions of a humanized or human anti-oxMIF binding domain described herein, such as the CDRs comprised in e.g. SEQ ID NOs: 9, 10, 11, 12 or 13 and/or one or more (e.g., all three) heavy chain complementary determining regions of a humanized or human anti-oxMIF binding domain described herein, e.g. SEQ ID NOs: 6, 7, or 8.

oxMIF binding specificity can be determined by any assay appropriate for determining selective binding to oxMIF, such as any competition assay against a control antibody, such as Imalumab, with respect to binding to oxMIF or various assays known in the art and as herein disclosed.

The term **"antibody"** herein is used in the broadest sense and encompasses polypeptides or proteins that consist of or comprise antibody domains, which are understood as constant and/or variable domains of the heavy and/or light chains of immunoglobulins, with or without a linker sequence. The term encompasses various antibody structures, including but not limited to monoclonal antibodies, polyclonal antibodies, multispecific antibodies such as bispecific antibodies, trispecific antibodies, and antibody fragments as long as they exhibit the desired antigen-binding activity, i.e. binding to oxMIF. The term also encompasses fusion proteins, such as fusions with immunotoxins or antibody conjugates, such as antibody drug conjugates binding to oxMIF.

Antibody domains may be of native structure or modified by mutagenesis or derivatization, e.g. to modify the antigen binding properties or any other property, such as stability or functional properties, such as binding to the Fc receptors, such as FcRn and/or Fc-gamma receptor. Polypeptide sequences are considered to be antibody domains, if comprising a beta-barrel structure consisting of at least two beta-strands of an antibody domain structure connected by a loop sequence.

It is understood that the term "antibody" includes antigen binding derivatives and fragments thereof. A derivative is any combination of one or more antibody domains or antibodies of the invention and/ or a fusion protein in which any domain of the antibody of the invention may be fused at any position of one or more other proteins, such as other antibodies or antibody formats, e.g. a binding structure comprising CDR loops, a receptor polypeptide, but also ligands, scaffold proteins, enzymes, labels, toxins and the like.

The term "antibody" shall particularly refer to polypeptides or proteins that exhibit binding properties to the target antigen oxMIF.

An **"antibody fragment"** refers to a molecule other than an intact antibody that comprises an antigen binding portion of an intact antibody that binds the antigen to which the intact antibody binds. Examples of antibody fragments include but are not limited to Fv, Fab, Fab', Fab'-SH, single chain antibody molecules (e.g. scFv), Fab-scFv fusion, Fab-(scFv)₂, Fab-scFv-Fc, Fab-(scFv)₂-Fc, F(ab')₂, ScFv-Fc, diabodies, cross-Fab fragments; linear antibodies; and multispecific antibodies formed from antibody fragments. In addition, antibody fragments comprise single chain polypeptides having the characteristics of a VH domain, namely being able to assemble together with a VL domain, or of a VL domain, namely being able to assemble together with a VH domain to a functional antigen binding site and thereby providing the antigen binding property of full-length antibodies. Antibody fragments as referred herein also encompass Fc domains comprising one or more structural loop regions containing antigen binding regions such as Fcab^{™} or full-length antibody formats with IgG structures in which the Fc region has been replaced by an Fcab^{™} containing second distinct antigen binding site.

The term **"functional variant"** or "functionally active variant" also includes naturally occurring allelic variants, as well as mutants or any other non-naturally occurring variants. As is known in the art, an allelic variant, or also referred to as homologue, is an alternate form of a nucleic acid or peptide that is characterized as having a substitution, deletion, or addition of one or more nucleotides or amino acids that does essentially not alter the biological function of the nucleic acid or polypeptide. Specifically, a functional variant may comprise a substitution, deletion and/or addition of 1, 2, 3, 4, 5, 6, 7, 8, 9, 10, 11, 12, 13, 14, 15, 16, 17, 18, 19 or 20 amino acid residues, or a combination thereof, which substitutions, deletions and/or additions are conservative modifications and do not alter the antigen binding properties. Specifically, a functional variant as described herein comprises no more than or up to 1, 2, 3, 4, 5, 6, 7, 8, 9, 10, 11, 12, 13, 14, 15, 16, 17, 18, 19 or 20 amino acid substitutions, deletions and/or additions, which are conservative modifications and do not alter the antibody's function. Specifically, a functionally active variant as described herein comprises up to 15, preferably up to 10 or 5, amino acid substitutions, deletions and/or additions, which are conservative modifications and do not alter the antibody's function.

Functional variants may be obtained by sequence alterations in the polypeptide or the nucleotide sequence, e.g. by one or more point mutations, wherein the sequence alterations retain or improve a function of the unaltered polypeptide or the nucleotide sequence, when used in combination of the invention. Such sequence alterations can include, but are not limited to, (conservative) substitutions, additions, deletions, mutations and insertions. Conservative substitutions are those that take place within a family of amino acids that are related in their side chains and chemical properties. Examples of such families are amino acids with basic side chains, with acidic side chains, with non-polar aliphatic side chains, with non-polar aromatic side chains, with uncharged polar side chains, with small side chains, with large side chains etc.

A point mutation is particularly understood as the engineering of a polynucleotide that results in the expression of an amino acid sequence that differs from the non-engineered amino acid sequence in the substitution or exchange, deletion or insertion of one or more single (non-consecutive) or doublets of amino acids for different amino acids.

As used herein, **"Fab fragment or Fab"** refers to an antibody fragment comprising a light chain fragment comprising a VL domain and a constant domain of a light chain (CL), and a VH domain and a first constant domain (CH1) of a heavy chain. The antibodies of the invention can comprise at least one Fab fragment, wherein either the variable regions or the constant regions of the heavy and light chain are exchanged. Due to the exchange of either the variable regions or the constant regions, said Fab fragment is also referred to as "cross-Fab fragment" or "crossover Fab fragment". Two different chain compositions of a crossover Fab molecule are possible and comprised in the antibodies of the invention: The variable regions of the Fab heavy and light chain can be exchanged, i.e. the crossover Fab molecule comprises a peptide chain composed of the light chain variable region (VL) and the heavy chain constant region (CH1), and a peptide chain composed of the heavy chain variable region (VH) and the light chain constant region (CL). This crossover Fab molecule is also referred to as CrossFab(VLVH). When the constant regions of the Fab heavy and light chain are exchanged, the crossover Fab molecule can comprise a peptide chain composed of the heavy chain variable region (VH) and the light chain constant region (CL), and a peptide chain composed of the light chain variable region (VL) and the heavy chain constant region (CH1). This crossover Fab molecule is also referred to as CrossFab(CLCH1).

A **"(scFv)2"** refers to an artificial monoclonal antibody which is a fusion protein consisting of two single-chain variable fragments (scFvs) of either different antibodies or the same antibody, or from four or two different genes, on a single peptide chain of about 50 kilodaltons.

A **"bs(scFv)2"** refers to an artificial monoclonal antibody which is a fusion protein consisting of two single-chain variable fragments (scFvs) of antibodies with different target antigens, or amino acid sequences from four different genes, on a single peptide chain of about 50 kilodaltons

A **"single chain Fab fragment"** or **"scFab"** is a polypeptide consisting of an antibody heavy chain variable domain (VH), an antibody constant domain 1 (CH1), an antibody light chain variable domain (VL), an antibody light chain constant domain (CL) and a linker, wherein said antibody domains and said linker can have the following orders in N-terminal to C-terminal direction: VH-CH1-linker-VL-CL, VL-CL-linker-VH-CH1, VH-CL-linker-VL-CH1 or VL-CH1-linker-VH-CL; and wherein said linker is a polypeptide of at least 20 amino acids, at least 30 amino acids, specifically between 32 and 50 amino acids. Said single chain Fab fragments VH-CH1-linker-VL-CL, VL-CL-linker-VH-CH1, VH-CL-linker-VL-CH1 and VL-CH1-linker-VH-CL, can be stabilized via the natural disulfide bond between the CL domain and the CH1 domain. In addition, these single chain Fab molecules might be further stabilized by generation of interchain disulfide bonds via insertion of cysteine residues.

The term "N-terminus" denotes the last amino acid of the N-terminus.

The term "C-terminus" denotes the last amino acid of the C-terminus.

A **"BiTE"** of "bi-specific T-cell engager" refers to an artificial monoclonal antibody which is a fusion protein consisting of two single-chain variable fragments (scFvs) of different antibodies, or amino acid sequences from four different genes, on a single peptide chain of about 50 kilodaltons. One of the scFvs can for example, but not limited to, bind to a T cell, e.g. via CD3, and the other to a tumor cell via oxMIF. Specifically, the BiTE is of about 50kDa.

The term **"minibody"** refers to an antibody which is composed of a pair of single-chain Fv fragments which are linked via CH3 domains (single chain Fv-CH3), and Fvs with distinct specificity, which paired to the former part through heterodimerization process (Hu S.Z. et al., 1996, Cancer Research, 56, 3055-3061). To promote the heterodimerization efficiency, single-residue mutations can be introduced into each CH3 domains to achieve a "knobs-into-holes" approach. Far more than that, additional cysteine residues can also be introduced into CH3 domains to stabilize the bispecific minibody structure. A version of a minibody, TriBi minibody, comprises a chain, which is designed to recognize antigens via its two Fv fragments, while the other chain possessing a Fv fragment takes charge of recruiting effector cells, such as T cytotoxic cells or NK cells. With the addition of this extra binding domain, the avidity of TriBi minibody is higher than that of the bispecific minibody. Specifically, the minibody is of about 75kDa.

The term "**DART**^{®}" refers to dual-affinity re-targeting antibodies which are the simplest form of bispecific antibodies (BsAb). A DART molecule consists of two engineered Fv fragments which have their own VH exchanged with the other one. The Fv1 is consisted of a VH from antibody A and a VL from antibody B, while the Fv2 is consisted of VH from Ab-B and VL from Ab-A. This inter-exchange of Fv domains releases variant fragments from the conformational constraint by the short linking peptide.

The term **"DutaMab^{™}"** refers to a format of BsAbs, which is formed by linking two independent paratopes in a single immunoglobulin chain.

The term **"TandAb^{®}"** or "tandem antibody" refers to antibodies having in tandem two VLs/VHs pairs from two distinct Fv, which also means tandem antibodies do not carry Fc domains. TandAbs are smaller than whole IgGs or IgG-derived bispecific Abs but larger than single domain bispecific Abs. The moderate size also endows TandAb an increased tissue penetrating ability and longer serum half-life. In addition, the tetravalent property, which means bivalent for each antigen, can improve its binding efficiency and consequent therapeutic outcome. Specifically, the TandAb is of about 100kDa.

The term **"diabody"** refers to a noncovalent dimer of single-chain Fv (scFv) fragment that consists of the heavy chain variable (V_{H}) and light chain variable (V_{L}) regions connected by a small peptide linker. Another form of diabody is single-chain (Fv)₂ in which two scFv fragments are covalently linked to each other. In addition, by tandem linking genes in each chain with internal linker, four VH and VL domains can be expressed in tandem and folded as single chain diabody (scDb), which is also an effective strategy for bispecific antibody production. Furthermore, fusing recombinant variable domains to an Fc region or CH3 domain (scDb-Fc and scDb-CH3, diabody-CH3) can double the valency of the final product. The increased size can also prolong the half-life of diabody in serum. Specifically, the diabody-CH3 is of about 125kDa.

The term "**crossMab**^{®}" (where mab refers to monoclonal antibody) is a format of bispecific Abs derived from independent parental antibodies. Heavy chain mispairing is avoided by applying the knobs-into-holes (KIH) method. Light chain mispairing is avoided as the bispecific antibody is produced with antibody domain exchange whereas either the variable domains or the constant domains (CL and CH1) of one Fab arm are swapped between the light and heavy chains. This "crossover" keeps the antigen-binding affinity and also preserves the two different arms in order to avoid light-chain mispairing. Examples of CrossMabs can be, but are not limited to Fab, VH-VL and CH1-CL exchanged in different regions. In CrossMAbs Fabs the full VH-CH1 and VL-CL regions are exchanged; in CrossMAb VH-VL format only the VH and VL regions are exchanged; in CrossMAb CH1-CL1 format the CH1 and CL regions of bispecific antibody are exchanged. Specifically, the CrossMab is of about 150kDa.

The term **"IgG-scFv"** refers to a kind of bispecific antibodies which is engineered for bispecificity by fusing two scFvs respectively to a monospecific IgG. The specificity of each scFv can be same or different. Furthermore, either the amino or the C terminus of each light or heavy chain can be appended with paired antibody variable domains, which leads to the production of diverse types of IgG-scFv BsAbs: IgG(H)-scFv or scFv-(H)IgG: IgG(H)-scFv, two scFvs with same specificity linked to the C terminus of the full-length IgG HC; scFv-(H)IgG, which is same like IgG(H)-scFv, except that the scFvs are linked to the HC N terminus. IgG(L)-scFv or scFv-(L)IgG: the two same scFvs connected to the C or N terminus of the IgG light chain, which forms the IgG(L)-scFv or scFv-(L)IgG, respectively. 2scFv-IgG or IgG-2scFv: generated by fusing two paired scFvs with different specificity to either the N terminus (2scFv-lgG) or the C terminus (IgG-2scFv). Specifically, the IgG(H)-scFv is about 200kDa.

According to a specific embodiment, the antibodies described herein may comprise one or more tags for purification and/or detection, such as but not limited to affinity tags, solubility enhancement tags and monitoring tags.

Specifically, the affinity tag is selected from the group consisting of poly-histidine tag, poly-arginine tag, peptide substrate for antibodies, chitin binding domain, RNAse S peptide, protein A, β-galactosidase, FLAG tag, Strep II tag, streptavidin-binding peptide (SBP) tag, calmodulin-binding peptide (CBP), glutathione S-transferase (GST), maltose-binding protein (MBP), S-tag, HA tag, and c-Myc tag, specifically the tag is a His tag comprising one or more H, such as a hexahistidine tag.

By **"fused"** or **"connected"** is meant that the components (e.g. a Fab molecule and an Fc domain subunit) are linked by peptide bonds, either directly or via one or more peptide linkers.

The term **"linker"** as used herein refers to a peptide linker and is preferably a peptide with an amino acid sequence with a length of 2, 3, 4, 5, 6, 7 or more amino acids, preferably with a length of 2-10, more preferably of 3-5 amino acids.

The term **"immunoglobulin"** refers to a protein having the structure of a naturally occurring antibody. For example, immunoglobulins of the IgG class are heterotetrameric glycoproteins of about 150,000 daltons, composed of two light chains and two heavy chains that are disulfide-bonded. From N- to C-terminus, each heavy chain has a variable region (VH), also called a variable heavy domain or a heavy chain variable domain, followed by three constant domains (CH1, CH2, and CH3), also called a heavy chain constant region. Similarly, from N- to C-terminus, each light chain has a variable region (VL), also called a variable light domain or a light chain variable domain, followed by a constant light (CL) domain, also called a light chain constant region. An immunoglobulin of the IgG class essentially consists of two Fab molecules and an Fc domain, linked via the immunoglobulin hinge region. The heavy chain of an immunoglobulin may be assigned to one of five types, called α (IgA), δ (IgD), ε (IgE), γ (IgG), or µ (IgM), some of which may be further divided into subtypes, e.g. γ₁ (IgG₁), γ₂ (IgG₂), γ₃ (IgG₃), γ₄ (IgG₄), α₁ (IgA₁) and α₂ (IgA₂). The light chain of an immunoglobulin may be assigned to one of two types, called kappa (κ) and lambda (λ).

The term **"chimeric antibody"** refers to an antibody in which a portion of the heavy and/or light chain is derived from a particular source or species, while the remainder of the heavy and/or light chain is derived from a different source or species, usually prepared by recombinant DNA techniques. Chimeric antibodies may comprise a rabbit or murine variable region and a human constant region. Chimeric antibodies are the product of expressed immunoglobulin genes comprising DNA segments encoding immunoglobulin variable regions and DNA segments encoding immunoglobulin constant regions. Methods for producing chimeric antibodies involve conventional recombinant DNA and gene transfection techniques are well known in the art (Morrison, S.L., et al., 1984).

A **"human antibody"** possesses an amino acid sequence which corresponds to that of an antibody produced by a human or a human cell or derived from a non-human source that utilizes human antibody repertoires or other human antibody-encoding sequences. This definition of a human antibody specifically excludes a humanized antibody comprising non-human antigen-binding residues. As also mentioned for chimeric and humanized antibodies, the term "human antibody" as used herein also comprises such antibodies which are modified in the constant region e.g. by "class switching" i.e. change or mutation of Fc parts (e.g. from IgG1 to IgG4 and/or IgG1/IgG4 mutation.)

The term **"recombinant human antibody",** as used herein, is intended to include all human antibodies that are prepared, expressed, created or isolated by recombinant means, such as antibodies isolated from a host cell such as a HEK cell, NS0 or CHO cell or from an animal (e.g. a mouse) that is transgenic for human immunoglobulin genes or antibodies expressed using a recombinant expression vector transfected into a host cell. The amino acid sequences of the VH and VL regions of the recombinant antibodies are sequences that, while derived from and related to human germ line sequences, may not naturally exist within the human antibody repertoire in vivo.

A **"human consensus framework"** is a framework which represents the most commonly occurring amino acid residues in a selection of human immunoglobulin VL or VH framework sequences. Generally, the selection of human immunoglobulin VL or VH sequences is from a subgroup of variable domain sequences. Generally, the subgroup of sequences is a subgroup as described in Kabat et al., 1991.

A **"humanized"** antibody refers to a chimeric antibody comprising amino acid residues from non-human HVRs and amino acid residues from human framework regions (FRs) which has undergone humanization. In certain embodiments, a humanized antibody will comprise substantially all of at least one, and typically two, variable domains, in which all or substantially all of the HVRs (e.g., CDRs) correspond to those of a non-human antibody, and all or substantially all of the FRs correspond to those of a human antibody. A humanized antibody optionally may comprise at least a portion of an antibody constant region derived from a human antibody. Other forms of humanized antibodies encompassed by the present invention are those in which the constant region has been additionally modified or changed from that of the original antibody to generate the new properties, e.g. in regard to C1q binding and/or Fc receptor (FcR) binding.

The term **"bispecific"** as used herein shall refer to the binding reaction at least with an oxMIF antigen and a further antigen, such as but not limited to e.g. CD3 antigen. A bispecific antibody specifically can comprise at least two sites with specific binding properties, wherein two different target antigens, oxMIF and a further antigen, are recognized by the antibody. An exemplary bispecific antibody format may comprise two binding sites, wherein each of the binding sites is capable of specifically binding a different antigen, e.g. CD3 and oxMIF. A further exemplary bispecific format may comprise more than two binding sites, wherein one or more binding sites bind to oxMIF and one or more binding sites are capable of specifically binding one or more different antigens, e.g. CD3.

**"Bispecific antibodies"** according to the invention are antibodies which have two different binding specificities. Bispecific antibodies can be prepared as full-length antibodies or antibody fragments as described herein. Immunoglobulin Fc heterodimers may be engineered through modifications to the CH3 domain interface, with different mutations on each domain such that the engineered Fc fragments, carrying the CH3 variant pair, preferentially form heterodimers rather than homodimers (Ha J-H. et al., 2016). Examples of bispecific antibody formats can be, but are not limited to bispecific IgGs (BsIgG), IgGs appended with an additional antigen-binding moiety, BsAb fragments, bispecific fusion proteins, BsAb conjugates, hybrid bsIgGs, variable domain only bispecific antibody molecules, CH1/CL fusion proteins, Fab fusion proteins, modified Fc and CH3 fusion proteins, appended IgGs-HC fusions, appended IgGs-LC fusions, appended IgGs-HC and LC fusions, Fc fusions, CH3 fusions, IgE/IgM CH2 fusions, F(ab')₂ fusions, CH1/CL, modified IgGs, non-immunoglobulin fusion proteins, Fc-modified IgGs, diabodies, etc. as described in Spiess C. et al., 2015, and Brinkmann U. and Kontermann R.E., 2017.

The oxMIF antibody of the invention may further comprise at least one binding site specifically recognizing a further epitope, e.g. of CD3, specifically an epitope of human CD3, including the CD3γ (gamma) chain, CD3δ (delta) chain, and two CD3ε (epsilon) chains which are present on the cell surface. Clustering of CD3 on T cells, such as by immobilized anti-CD3 antibodies leads to T cell activation similar to the engagement of the T cell receptor but independent of its clone-typical specificity. In certain embodiments, the CD3 binding domain of the antibody described herein exhibits not only potent CD3 binding affinities with human CD3, but shows also excellent cross-reactivity with the respective cynomolgus monkey CD3 proteins. In some instances, the CD3 binding domain of the antibody is cross-reactive with CD3 from cynomolgus monkey. In one embodiment, the anti-CD3 binding site comprises one or more (e.g., all three) light chain complementary determining regions of an anti-CD3 binding domain described herein, and/or one or more (e.g., all three) heavy chain complementary determining regions of an anti-CD3 binding domain described herein, e.g., an anti-CD3 binding domain comprising one or more, e.g., all three, LC CDRs and one or more, e.g., all three, HC CDRs.

Specifically, the anti-oxMIF antibody comprises a second or further binding specificity which is directed to CD3 as described in WO2019234241A1. Such bispecific anti-oxMIF antibody may comprise a binding site specifically recognizing CD3 which comprises
(a) a heavy chain variable region comprising
   a CDR1-H2 sequence which comprises or has at least 70%, specifically 80%, 90%, 95% or 99% sequence identity to any of the sequences selected from the group consisting of RYTMH (SEQ ID NO:14), GYGMH (SEQ ID NO:15) and SFPMA (SEQ ID NO:16), and
   a CDR2-H2 which comprises, or has at least 70%, specifically 80%, 90%, 95% or 99% sequence identity to, any of the sequences selected from the group consisting of YINPSRGYTNYNQKFKD (SEQ ID NO:17), VIWYDGSKKYYVDSVKG (SEQ ID NO:18), and TISTSGGRTYYRDSVKG (SEQ ID NO:19), and
   a CDR3-H2 which has at least 70%, specifically 80%, 90%, 95% or 99% sequence identity to any of the sequences selected from the group consisting of YYDDHYCLDY (SEQ ID NO:20), QMGYWHFDL (SEQ ID NO:21), FRQYSGGFDY (SEQ ID NO:22), and YYDDHYSLDY (SEQ ID NO:23), and
(b) a light chain variable region comprising
   a CDR1-L2 which comprises, or has at least 70%, specifically 80%, 90%, 95% or 99% sequence identity to, any of the sequences selected from the group consisting of RASSSVSYMN (SEQ ID NO:24), SASSSVSYMN (SEQ ID NO:25), RASQSVSSYLA (SEQ ID NO:26) and TLSSGNIENNYVH (SEQ ID NO:27), and
   a CDR2-L2 which comprises, or has at least 70%, specifically 80%, 90%, 95% or 99% sequence identity to, any of the sequences selected from the group consisting of DTSKVAS (SEQ ID N: 28), DTSKLAS (SEQ ID:NO 29), DASNRAT (SEQ ID NO:30) and DDDKRPD (SEQ ID NO:31), and
   a CDR3-L2 which comprises, or has at least 70%, specifically 80%, 90%, 95% or 99% sequence identity to, any of the sequences selected from the group consisting of QQWSSNPLT (SEQ ID NO:32), QQWSSNPFT (SEQ ID NO:33), QQRSNWPPLT (SEQ ID NO:34), SQWLYGMDV (SEQ ID NO:35), and QQWSSNP (SEQ ID NO:36).

More specifically a bispecific anti-oxMIF/anti-CD3 antibody comprises 0, 1, or 2 point mutations in each of the CDR sequences listed above.

A further specific embodiment refers to an anti-oxMIF/anti-CD3 bs(scFv)2 wherein the corresponding variable heavy chain regions (VH) and the corresponding variable light chain regions (VL) regions are arranged, from N-terminus to C- terminus, in the order, VH(oxMIF)-V_{L}(oxMIF)-VH(CD3)-VL(CD3), VH(CD3)-VL(CD3)-VH(oxMIF)-VL(oxMIF) or VH(CD3)-VL(CD3)-VL(oxMIF)-VH(oxMIF).

In a further embodiment, the antibody is a bispecific antibody, specifically selected from the group consisting of bispecific IgG, IgG appended with a CD3 binding site, BsAb fragments, bispecific fusion proteins, BsAb conjugates.

The term **"antigen"** as used herein interchangeably with the terms **"target"** or **"target antigen"** shall refer to a whole target molecule or a fragment of such molecule recognized by an antibody binding site. Specifically, substructures of an antigen, e.g. a polypeptide or carbohydrate structure, generally referred to as "epitopes", e.g. B-cell epitopes or T-cell epitope, which are immunologically relevant, may be recognized by such binding site.

The term **"epitope"** as used herein shall in particular refer to a molecular structure which may completely make up a specific binding partner or be part of a specific binding partner to a binding site of an antibody format of the present invention. An epitope may either be composed of a carbohydrate, a peptidic structure, a fatty acid, an organic, biochemical or inorganic substance or derivatives thereof and any combinations thereof. If an epitope is comprised in a peptidic structure, such as a peptide, a polypeptide or a protein, it will usually include at least 3 amino acids, specifically 5 to 40 amino acids, and specifically less than 10 amino acids, specifically between 4 to 10 amino acids. Epitopes can be either linear or conformational epitopes. A linear epitope is comprised of a single segment of a primary sequence of a polypeptide or carbohydrate chain. Linear epitopes can be contiguous or overlapping. Conformational epitopes are comprised of amino acids or carbohydrates brought together by folding the polypeptide to form a tertiary structure and the amino acids are not necessarily adjacent to one another in the linear sequence. Such oxMIF epitope may be sequence EPCALCS (SEQ ID NO:43) located within the central region of oxMIF.

The term **"antigen binding domain"** or "binding domain" or "binding-site" refers to the part of an antigen binding moiety that comprises the area which specifically binds to and is complementary to part or all of an antigen. Where an antigen is large, an antigen binding molecule may only bind to a particular part of the antigen, which part is termed an epitope. An antigen binding domain may be provided by, for example, one or more antibody variable domains (also called antibody variable regions). Preferably, an antigen binding domain comprises an antibody light chain variable region (VL) and an antibody heavy chain variable region (VH).

The term **"binding site"** as used herein with respect to the antibody of the present invention refers to a molecular structure capable of binding interaction with an antigen. Typically, the binding site is located within the complementary determining region (CDR) of an antibody, herein also called "a CDR binding site", which is a specific region with varying structures conferring binding function to various antigens. The varying structures can be derived from natural repertoires of antibodies, e.g. murine or human repertoires, or may be recombinantly or synthetically produced, e.g. by mutagenesis and specifically by randomization techniques. These include mutagenized CDR regions, loop regions of variable antibody domains, in particular CDR loops of antibodies, such as CDR1, CDR2 and CDR3 loops of any of VL and/or VH antibody domains. The antibody format as used according to the invention typically comprises one or more CDR binding sites, each specific to an antigen.

The term **"specific"** as used herein shall refer to a binding reaction which is determinative of the cognate ligand of interest in a heterogeneous population of molecules. Herein, the binding reaction is at least with an oxMIF antigen. Thus, under designated conditions, e.g. immunoassay conditions, the antibody that specifically binds to its particular target does not bind in a significant amount to other molecules present in a sample, specifically it does not show substantial cross-reactivity to reduced MIF.

A specific binding site is typically not cross-reactive with other targets. Still, the specific binding site may specifically bind to one or more epitopes, isoforms or variants of the target, or be cross-reactive to other related target antigens, e.g., homologs or analogs.

The specific binding means that binding is selective in terms of target identity, high, medium or low binding affinity or avidity, as selected. Selective binding is usually achieved if the binding constant or binding dynamics to a target antigen such as oxMIF is at least 10-fold different, preferably the difference is at least 100-fold, and more preferred a least 1000-fold compared to binding constant or binding dynamics to an antigen which is not the target antigen.

The term **"valent"** as used within the current specification denotes the presence of a specified number of binding sites in an antibody molecule. As such, the terms "bivalent", "tetravalent", and "hexavalent" denote the presence of two binding sites, four binding sites, and six binding sites, respectively, in an antibody molecule.

The term **"monovalent"** as used herein with respect to a binding site of an antibody shall refer to a molecule comprising only one binding site directed against a target antigen. The term "valency" is thus understood as the number of binding sites directed against the same target antigen, either specifically binding the same or different epitopes of an antigen.

The antibody of the present invention is understood to comprise a monovalent, bivalent, tetravalent or multivalent binding site specifically binding oxMIF

According to a further embodiment, the antibody can comprise one or more additional binding sites specifically recognizing one or more antigens expressed on effector T cells, NK-cells or macrophages, specifically one or more of CD3, ADAM17, CD2, CD4, CD5, CD6, CD8, CD11a, CD11b, CD14, CD16, CD16b, CD25, CD28, CD30, CD32a, CD40, CD 40L, CD44, CD45, CD56, CD57, CD64, CD69, CD74, CD89, CD90, CD137, CD177, CEAECAM6, CEACAM8, HLA-Dra cahin, KIR, LSECtin or SLC44A2.

According to an alternative embodiment, the antibody of the invention is a bispecific antibody further comprising one or more of the CD3 variable binding domains of mosunetuzumab, pasotuxizumab, cibisatamab, otelixizumab, teplizumab, visilizumab or foralumab.

According to a specific embodiment, the anti-CD3 binding site comprises complementary determining regions (CDRs) selected from the group consisting of muromonab-CD3 (OKT3), otelixizumab (TRX4), teplizumab (MGA031), visilizumab (Nuvion), solitomab, blinatumomab, pasotuxizumab, cibisatamab, mosunetuzumab,

SP34, X35, VIT3, BMA030 (BW264/56), CLB-T3/3, CRIS7, YTH12.5, F111-409, CLB-T3.4.2, TR-66, WT32, SPv-T3b, 11D8, XIII-141, XIII-46, XIII-87, 12F6, T3/RW2-8C8, T3/RW2-4B6, OKT3D, M-T301, SMC2, F101.01, UCHT-1 and WT-31 and any humanized derivatives thereof, if applicable.

Specifically, one or more CDRs of anti-CD3 variable regions or CDRs comprising or having at least 70%, specifically 80%, 90%, 95% or 99% sequence identity to any of the CDR sequences of muromonab, otelixizumab, teplizumab, visilizumab, solitomab, blinatumomab, pasotuxizumab, cibisatamab, mosunetuzumab may be used as CD3 binding domains according to the invention.

The term **"hypervariable region"** or "HVR," as used herein refers to each of the regions of an antibody variable domain which are hypervariable in sequence and/or form structurally defined loops ("hypervariable loops"). Generally, native four-chain antibodies comprise six HVRs; three in the VH (H1, H2, H3), and three in the VL (L1, L2, L3). HVRs generally comprise amino acid residues from the hypervariable loops and/or from the "complementarity determining regions" (CDRs), the latter being of highest sequence variability and/or involved in antigen recognition (Kabat et al., 1991) Hypervariable regions (HVRs) are also referred to as complementarity determining regions (CDRs), and these terms are used herein interchangeably in reference to portions of the variable region that form the antigen binding regions. The exact residue numbers which encompass a particular CDR will vary depending on the sequence and size of the CDR. Those skilled in the art can routinely determine which residues comprise a particular CDR given the variable region amino acid sequence of the antibody.

Kabat defined a numbering system for variable region sequences that is applicable to any antibody. One of ordinary skill in the art can unambiguously assign this system of "Kabat numbering" to any variable region sequence, without reliance on any experimental data beyond the sequence itself. The Kabat numbering of residues can be determined for a given antibody by alignment at regions of homology of the sequence of the antibody with a "standard" Kabat numbered sequence. As used herein, "Kabat numbering" refers to the numbering system set forth by Kabat et al., 1983, U.S. Dept. of Health and Human Services, "Sequence of Proteins of Immunological Interest". Unless otherwise specified, references to the numbering of specific amino acid residue positions in an antibody variable region are according to the Kabat numbering system. The numbering of the constant region is according to EU numbering index.

CDRs also comprise "specificity determining residues," or "SDRs," which are residues that contact antigen. SDRs are contained within regions of the CDRs called abbreviated-CDRs, or a-CDRs. Unless otherwise indicated, HVR residues and other residues in the variable domain (e.g., FR residues) are numbered herein according to Kabat et al., *supra.*

A **"point mutation"** is particularly understood as the engineering of a polynucleotide that results in the expression of an amino acid sequence that differs from the non-engineered amino acid sequence in the substitution or exchange, deletion or insertion of one or more single (non-consecutive) or doublets of amino acids for different amino acids. Preferred point mutations refer to the exchange of amino acids of the same polarity and/or charge. In this regard, amino acids refer to twenty naturally occurring amino acids encoded by sixty-one triplet codons. These 20 amino acids can be split into those that have neutral charges, positive charges, and negative charges.

**"Percent (%) sequence identity"** with respect to the polypeptide sequences identified herein is defined as the percentage of amino acid residues in a candidate sequence that are identical with the amino acid residues in the specific polypeptide sequence, after aligning the sequence and introducing gaps, if necessary, to achieve the maximum percent sequence identity, and not considering any conservative substitutions as part of the sequence identity. Those skilled in the art can determine appropriate parameters for measuring alignment, including any algorithms needed to achieve maximal alignment over the full length of the sequences being compared.

According to the present invention, sequence identity of the variable or constant region sequences is at least 95%, 96%, 97%, 98%, 99%, 99.5% or 100% with the respective sequences described herein.

A **"subject"** is a mammal. Mammals include, but are not limited to, domesticated animals (e.g., cows, sheep, cats, dogs, and horses), primates (e.g., humans and non-human primates such as monkeys), rabbits, and rodents (e.g., mice and rats). In certain embodiments, the individual or subject is a human.

An **"isolated" nucleic acid"** refers to a nucleic acid molecule that has been separated from a component of its natural environment. An isolated nucleic acid includes a nucleic acid molecule contained in cells that ordinarily contain the nucleic acid molecule, but the nucleic acid molecule is present extrachromosomally or at a chromosomal location that is different from its natural chromosomal location.

**"Isolated nucleic acid encoding an anti-oxMlF** " refers to one or more nucleic acid molecules encoding antibody heavy and light chains (or fragments thereof), including such nucleic acid molecule(s) in a single vector or separate vectors, and such nucleic acid molecule(s) present at one or more locations in a host cell.

**"No substantial cross-reactivity"** means that a molecule (*e.g*., an antibody) does not recognize or specifically bind an antigen different from the actual target antigen of the molecule (e.g. an antigen closely related to the target antigen), specifically reduced MIF, particularly when compared to that target antigen. For example, an antibody may bind less than about 10% to less than about 5% to an antigen different from the actual target antigen, or may bind said antigen different from the actual target antigen at an amount consisting of less than about 10%, 9%, 8% 7%, 6%, 5%, 4%, 3%, 2%, 1%, 0.5%, 0.2%, or 0.1%, preferably less than about 2%, 1%, or 0.5%, and most preferably less than about 0.2% or 0.1% to an antigen different from the actual target antigen. Binding can be determined by any method known in the art such as, but not limited to ELISA or surface plasmon resonance.

The recombinant production of the antibody of the invention preferably employs an expression system, e.g. including expression constructs or vectors comprising a nucleotide sequence encoding the antibody format.

The term **"expression system"** refers to nucleic acid molecules containing a desired coding sequence and control sequences in operable linkage, so that hosts transformed or transfected with these sequences are capable of producing the encoded proteins. In order to effect transformation, the expression system may be included on a vector; however, the relevant DNA may then also be integrated into the host chromosome. Alternatively, an expression system can be used for *in vitro* transcription/translation.

**"Expression vectors"** used herein are defined as DNA sequences that are required for the transcription of cloned recombinant nucleotide sequences, i.e. of recombinant genes and the translation of their mRNA in a suitable host organism. Expression vectors comprise the expression cassette and additionally usually comprise an origin for autonomous replication in the host cells or a genome integration site, one or more selectable markers (e.g. an amino acid synthesis gene or a gene conferring resistance to antibiotics such as zeocin, kanamycin, G418 or hygromycin), a number of restriction enzyme cleavage sites, a suitable promoter sequence and a transcription terminator, which components are operably linked together. The terms "plasmid" and "vector" as used herein include autonomously replicating nucleotide sequences as well as genome integrating nucleotide sequences.

Specifically, the term refers to a vehicle by which a DNA or RNA sequence (e.g. a foreign gene), e.g. a nucleotide sequence encoding the antibody format of the present invention, can be introduced into a host cell, so as to transform the host and promote expression (e.g. transcription and translation) of the introduced sequence. Plasmids are preferred vectors of the invention.

Vectors typically comprise the DNA of a transmissible agent, into which foreign DNA is inserted. A common way to insert one segment of DNA into another segment of DNA involves the use of enzymes called restriction enzymes that cleave DNA at specific sites (specific groups of nucleotides) called restriction sites.

A **"cassette"** refers to a DNA coding sequence or segment of DNA that code for an expression product that can be inserted into a vector at defined restriction sites. The cassette restriction sites are designed to ensure insertion of the cassette in the proper reading frame. Generally, foreign DNA is inserted at one or more restriction sites of the vector DNA, and then is carried by the vector into a host cell along with the transmissible vector DNA. A segment or sequence of DNA having inserted or added DNA, such as an expression vector, can also be called a "DNA construct". A common type of vector is a "plasmid", which generally is a self-contained molecule of double-stranded DNA that can readily accept additional (foreign) DNA and which can readily be introduced into a suitable host cell. A vector of the invention often contains coding DNA and expression control sequences, e.g. promoter DNA, and has one or more restriction sites suitable for inserting foreign DNA. Coding DNA is a DNA sequence that encodes a particular amino acid sequence for a particular polypeptide or protein such as an antibody format of the invention. Promoter DNA is a DNA sequence which initiates, regulates, or otherwise mediates or controls the expression of the coding DNA. Promoter DNA and coding DNA may be from the same gene or from different genes, and may be from the same or different organisms. Recombinant cloning vectors of the invention will often include one or more replication systems for cloning or expression, one or more markers for selection in the host, e.g. antibiotic resistance, and one or more expression cassettes.

The procedures used to ligate DNA sequences, e.g. providing or coding for the factors of the present invention and/or the protein of interest, a promoter, a terminator and further sequences, respectively, and to insert them into suitable vectors containing the information necessary for integration or host replication, are well known to persons skilled in the art, e.g. described by Sambrook et al, 2012.

A **host cell** is specifically understood as a cell, a recombinant cell or cell line transfected with an expression construct, such as a vector according to the invention.

The term "**host cell line**" as used herein refers to an established clone of a particular cell type that has acquired the ability to proliferate over a prolonged period of time. The term host cell line refers to a cell line as used for expressing an endogenous or recombinant gene to produce polypeptides, such as the recombinant antibody format of the invention.

A **"production host cell"** or "production cell" is commonly understood to be a cell line or culture of cells ready-to-use for cultivation in a bioreactor to obtain the product of a production process, the recombinant antibody format of the invention. The host cell type according to the present invention may be any prokaryotic or eukaryotic cell.

The term **"recombinant"** as used herein shall mean "being prepared by genetic engineering" or "the result of genetic engineering", e.g. specifically employing heterologous sequences incorporated in a recombinant vector or recombinant host cell.

An antibody of the invention may be produced using any known and well-established expression system and recombinant cell culturing technology, for example, by expression in bacterial hosts (prokaryotic systems), or eukaryotic systems such as yeasts, fungi, insect cells or mammalian cells. An antibody molecule of the present invention may be produced in transgenic organisms such as a goat, a plant or a transgenic mouse, an engineered mouse strain that has large fragments of the human immunoglobulin loci and is deficient in mouse antibody production. An antibody may also be produced by chemical synthesis.

According to a specific embodiment, the host cell is a production cell line of cells selected from the group consisting of CHO, PerC6, CAP, HEK, HeLa, NS0, SP2/0, hybridoma and Jurkat. More specifically, the host cell is obtained from CHO cells.

The host cell of the invention is specifically cultivated or maintained in a serum-free culture, e.g. comprising other components, such as plasma proteins, hormones, and growth factors, as an alternative to serum.

Host cells are most preferred, when being established, adapted, and completely cultivated under serum free conditions, and optionally in media which are free of any protein/peptide of animal origin.

Anti-oxMlF antibodies of the invention can be recovered from the culture medium using standard protein purification methods.

The term **"pharmaceutical formulation"** refers to a preparation which is in such form as to permit the biological activity of an active ingredient contained therein to be effective, and which contains no additional components which are unacceptably toxic to a subject to which the formulation would be administered.

A **"pharmaceutically acceptable carrier"** refers to an ingredient in a pharmaceutical formulation, other than an active ingredient, which is nontoxic to a subject. Some examples of pharmaceutically acceptable carriers are water, saline, phosphate buffered saline, amino acids such as glycine or histidine, dextrose, glycerol, ethanol and the like, as well as combinations thereof. In many cases, it will be preferable to include isotonic agents, for example, sugars, polyalcohols such as mannitol, sorbitol, or sodium chloride in the composition. Additional examples of pharmaceutically acceptable substances are wetting agents or minor amounts of auxiliary substances such as wetting or emulsifying agents, preservatives or buffers, which enhance the shelf life or effectiveness of the antibody.

As used herein, **"treatment",** "treat" or "treating" refers to clinical intervention in an attempt to alter the natural course of the individual being treated, and can be performed either for prophylaxis or during the course of clinical pathology. Desirable effects of treatment include, but are not limited to, preventing occurrence or recurrence of disease, alleviation of symptoms, diminishment of any direct or indirect pathological consequences of the disease, preventing metastasis, decreasing the rate of disease progression, amelioration or palliation of the disease state, and remission or improved prognosis. In some embodiments, antibodies of the invention are used to delay development of a disease or to slow the progression of a disease.

The anti-oxMIF antibody of the invention and the pharmaceutical compositions comprising it, can be administered in combination with one or more other therapeutic, diagnostic or prophylactic agents. Additional therapeutic agents include other antineoplastic, antitumor, anti-angiogenic, chemotherapeutic agents, steroids, or checkpoint inhibitors depending on the disease to be treated.

The pharmaceutical compositions of this invention may be in a variety of forms, for example, liquid, semi-solid and solid dosage forms, such as liquid solutions (e.g., injectable and infusible solutions), dispersions or suspensions, tablets, pills, powders, liposomes and suppositories. The preferred form depends on the intended mode of administration and therapeutic application. Typical preferred compositions are in the form of injectable or infusible solutions, such as compositions similar to those used for passive immunization of humans. The preferred mode of administration is parenteral (e.g., intravenous, subcutaneous, intraperitoneal, intramuscular). In a preferred embodiment, the antibody is administered by intravenous infusion or injection. In another preferred embodiment, the antibody is administered by intramuscular or subcutaneous injection. As will be appreciated by the skilled artisan, the route and/or mode of administration will vary depending upon the desired results.

Due to the optimized and advantageous properties of the inventive antibody, high dosages of the antibody composition can be administered. Specifically, the composition comprises about 10 to 250 mg/ml of the antibody, specifically 25 to 100 mg/ml, specifically ≥ 50 mg/ml.

The anti-oxMIF antibody may be administered once, but more preferably is administered multiple times. For example, the antibody may be administered from three times daily to once every six months or longer. The administering may be on a schedule such as three times daily, twice daily, once daily, once every two days, once every three days, once weekly, once every two weeks, once every month, once every two months, once every three months and once every six months.

The invention also relates to compositions comprising an anti-oxMIF antibody or an antigen-binding portion thereof, for the treatment of a subject in need of treatment for MIF-related conditions, specifically immunological diseases such as inflammatory diseases and hyperproliferative disorders. In some embodiments, the subject in need of treatment is a human.

The term **"cancer"** as used herein refers to proliferative diseases, specifically to solid cancers, such as colorectal cancer, ovarian cancer, pancreas cancer, lung cancer, melanoma, squamous cell carcinoma (SCC) (e.g., head and neck, esophageal, and oral cavity), hepatocellular carcinoma, colorectal adenocarcinoma, kidney cancer, medullary thyroid cancer, papillary thyroid cancer, astrocytic tumor, neuroblastoma, Ewing's sarcoma, cervical cancer, endometrial carcinoma, breast cancer, prostate cancer, and malignant seminoma, including refractory versions of any of the above cancers, or a combination of one or more of the above cancers.

Hyperproliferative disorders, such as cancerous diseases or cancer, that may be treated by anti-oxMIF antibodies of the invention can involve any tissue or organ and include but are not limited to brain, lung, squamous cell, bladder, gastric, pancreatic, breast, head, neck, liver, renal, ovarian, prostate, colorectal, esophageal, gynecological, nasopharynx, or thyroid cancers, melanomas, lymphomas, leukemias or multiple myelomas. In particular, anti-oxMIF antibodies of the invention are useful to treat carcinomas of the ovary, pancreas, colon and lung.

In a specific embodiment, the antibodies highly suitable for the treatment of cancerous diseases, specifically for the treatment of solid tumors are recombinant anti-oxMIF antibodies or antigen binding fragments thereof having reduced aggregation potential and reduced hydrophobicity, comprising the following variable domains:
(a1) a light chain variable domain comprising SEQ ID NO:9 with 1, 2, 3, 4, or 5 amino acid substitutions selected from M30L, F49Y, A51G, P80S, W93F, or
(a2) a light chain variable domain comprising SEQ ID NO:9 with 1, 2, 3, 4, or 5 amino acid substitutions selected from M30L, F49Y, A51G, P80S and W93F, and with 1, 2, 3, 4, or 5 further amino acid substitutions, with the proviso that the tyrosine at position 36is preserved; and one of
(b1) a heavy chain variable domain comprising SEQ ID NO:6; or
(b2) a heavy chain variable domain comprising SEQ ID NO:6 with 1 or 2 amino acid substitutions selected from L5Q andW97Y, or
(b3) a heavy chain variable domain comprising SEQ ID NO:6 with 1 or 2 amino acid substitutions selected from L5Q and W97Y, and with 1, 2, 3, 4 or 5 further amino acid substitutions, wherein amino acid positions are numbered according to Kabat, and wherein the aggregation potential and hydrophobicity are reduced compared to an antibody comprising SEQ ID NO:6 and SEQ ID NO:9 lacking the amino acid substitutions.

Alternatively, the treatment of solid tumors can be performed with recombinant anti-oxMIF antibodies comprising a light chain variable domain comprising SEQ ID NO:9 or a light chain variable domain with at least 95% sequence identity to SEQ ID NO:9 comprising a tyrosine at position 36 and a heavy chain variable domain comprising SEQ ID NO:6 and amino acid substitutions W97Y and/or L5Q, or a heavy chain variable domain comprising SEQ ID NO:6 with 1, 2, 3, 4 or 5 amino acid substitutions and further comprising amino acid substitutions W97Y and/or L5Q,

In a further specific embodiment, the antibodies highly suitable for the treatment of cancerous diseases, specifically for the treatment of solid tumors, are recombinant anti-oxMIF antibodies described having also enhanced effector functions with a heavy chain constant region comprising
(a) SEQ ID NO:2 or a functional variant having at least 95% sequence identity to SEQ ID NO:2 and comprising amino acids 239D and 332E; or
(b) SEQ ID NO:3, or a functional variant having at least 95% sequence identity to SEQ ID NO:3 and comprising amino acids 239D, 268F, 324T and 332E; or
(c) SEQ ID NO:4, or a functional variant having at least 95% sequence identity to SEQ ID NO:4 and comprising amino acids 239D, and 332E; or
(d) SEQ ID NO:5, or a functional variant having at least 95% sequence identity to SEQ ID NO:5 and comprising amino acids 235V, 243L, 292P, 300L, and 396L.

The invention also encompasses a pharmaceutical composition comprising the antibody of the invention for use in methods for the treatment of inflammatory diseases such as vasculitis, arthritis, sepsis, septic shock, endotoxic shock, toxic shock syndrome, acquired respiratory distress syndrome, glomerulonephritis, inflammatory bowel disease, Crohn's disease, ulcerative colitis, peritonitis, nephritis, atopic dermatitis, asthma, conjunctivitis, fever, Malaria, NASH (nonalcoholic steatosis hepatis), multiple sclerosis, acute and chronic pancreatitis, Type 1 diabetes, IgA nephropathy, interstitial cystitis, post COVID syndrome, psoriasis, glomerulonephritis, inflammatory bowel disease, nephritis and peritonitis, systemic lupus erythematosus (SLE - including lupus nephritis), asthma, rheumatoid arthritis (RA) and inflammatory bowel disease (IBD) in a subject, including a human, comprising the step of administering to said subject in need thereof a therapeutically effective amount of an anti-oxMIF antibody or antigen-binding portion thereof.

In a specific embodiment, the antibodies highly suitable for the treatment of inflammatory or infectious diseases are recombinant anti-oxMIF antibodies or antigen binding fragments thereof having reduced aggregation potential and reduced hydrophobicity,
the following variable domains:
(a1) a light chain variable domain comprising SEQ ID NO:9 with 1, 2, 3, 4, or 5 amino acid substitutions selected from M30L, F49Y, A51G, P80S, and W93F, or
(a2) a light chain variable domain comprising SEQ ID NO:9 with 1, 2, 3, 4, or 5 amino acid substitutions, selected from M30L, F49Y, A51G, P80S and W93F, and with 1, 2, 3, 4, or 5 further amino acid substitutions, with the proviso that the tyrosine at position 36 is preserved, and one of
(b1) a heavy chain variable domain comprising SEQ ID NO:6; or
(b2) a heavy chain variable domain comprising SEQ ID NO:6 with 1 or 2 amino acid substitutions selected from L5Q and W97Y, or
(b3) a heavy chain variable domain comprising SEQ ID NO:6 1 or 2 amino acid substitutions selected from L5Q and W97Y, and with 1, 2, 3, 4 or 5 further amino acid substitutions; wherein amino acid positions are numbered according to Kabat and wherein the aggregation potential and hydrophobicity are reduced compared to an antibody comprising SEQ ID NO:6 and SEQ ID NO:9 lacking the amino acid substitutions.

Alternatively, the treatment of inflammatory or infectious diseases can be performed with recombinant anti-oxMIF antibodies comprising a light chain variable domain comprising SEQ ID NO:9 or a light chain variable domain with at least 95% sequence identity to SEQ ID NO:9 comprising a tyrosine at position 36 and a heavy chain variable domain comprising SEQ ID NO:6 and amino acid substitutions W97Y and/or L5Q, or a heavy chain variable domain comprising SEQ ID NO:6 with 1, 2, 3, 4 or 5 amino acid substitutions and further comprising amino acid substitutions W97Y and/or L5Q,

In a further specific embodiment, the antibodies highly suitable for the treatment of inflammatory or infectious diseases, are recombinant anti-oxMIF antibodies described having also increased preferential binding to inhibitory receptor FcyRIIB, or have hyper α 2,6- N-linked sialylation.

### EXAMPLES

The examples described herein are illustrative of the present invention and are not intended to be limitations thereon. Accordingly, it should be understood that the examples are illustrative only and are not limiting upon the scope of the invention.

### Example 1: Reduced aggregation propensity and reduced hydrophobicity of the newly designed antibodies.

**Table 1: Variant IDs of the newly designed antibodies and sequence combinations of molecules in examples**

| **Variant ID (Sample)** | **VH** | **VL** | **CH** | **CL** |
|---|---|---|---|---|
| C0064 | Wt (SEQ ID NO:6) | Wt (SEQ ID NO:9) | S239D/I332E (SEQ ID NOS) | SEQ ID NO:38 |
| C0065 | Wt (SEQ ID NO:6) | Wt (SEQ ID NO:9) | S239D/I332E/H268F/S324T (SEQ ID NO:3) | SEQ ID NO:38 |
| C0067 | Wt (SEQ ID NO:6) | Wt (SEQ ID NO:9) | L235V/F243L/R292P/Y300L/ P396L/E356D/M358L (SEQ ID NO:5) | SEQ ID NO:38 |
| C0097 | Wt (SEQ ID NO:6) | F49Y/A51G/W93F (SEQ ID NO:11) | S239D/I332E (SEQ ID NO:2) | SEQ ID NO:38 |
| C0098 | Wt (SEQ ID NO:6) | F49Y/A51G/W93F (SEQ ID NO:11) | S239D/I332E/268F/S324T (SEQ ID NO:3) | SEQ ID NO:38 |
| C0099 | Wt (SEQ ID NO:6) | F49Y/A51G/W93F (SEQ ID NO:11) | R214K/S239D/K274Q/Y296F /Y300F/L309V/I332E/A339T/ V397M (SEQ ID NO:4) | SEQ ID NO:38 |
| C0100 | Wt (SEQ ID NO:6) | F49Y/A51G/W93F (SEQ ID NO:11) | L235V/F243L/R292P/Y300L/ P396L/E356D/M358L (SEQ ID NO:5) | SEQ ID NO:38 |
| C0108 | L5Q/W97Y (SEQ ID NO:8) | F49Y/A51G/W93F (SEQ ID NO:11) | R214K/S239D/K274Q/Y296F /Y300F/L309V/I332E/A339T/ V397M (SEQ ID NO:4) | SEQ ID NO:38 |
| C0109 | L5Q/W97Y (SEQ ID NO:8) | F49Y/A51G/M30L/ W93F/P80S (SEQ ID NO:13) | R214K/S239D/K274Q/Y296F /Y300F/L309V/I332E/A339T/ V397M (SEQ ID NO:4) | SEQ ID NO:38 |
| C0008 (control antibody) | Wt (SEQ ID NO:6) | Wt (SEQ ID NO:9) | Wt (SEQ ID NO:1) | SEQ ID NO:38 |
| C0070 | L5Q/W97Y (SEQ ID NO:8) | Wt (SEQ ID NO:9) | Wt (SEQ ID NO:1) | SEQ ID NO:38 |
| C0073 | Wt (SEQ ID NO:6) | F49Y/A51G/W93F (SEQ ID NO:11) | Wt (SEQ ID NO:1) | SEQ ID NO:38 |
| C0076 | L5Q (SEQ ID NO:7) | F49Y/A51G (SEQ ID NO:10) | Wt (SEQ ID NO:1) | SEQ ID NO:38 |
| C0077 | L5Q (SEQ ID NO:7) | Y36F/F49Y/A51G (SEQ ID NO:37) | Wt (SEQ ID NO:1) | SEQ ID NO:38 |
| C0078 | L5Q (SEQ ID NO:7) | F49Y/A51G/W93F (SEQ ID NO:11) | Wt (SEQ ID NO:1) | SEQ ID NO:38 |
| C0081 | L5Q/W97Y (SEQ ID NO:8) | F49Y/A51G (SEQ ID NO:10) | Wt (SEQ ID NO:1) | SEQ ID NO:38 |
| C0083 | L5Q/W97Y (SEQ ID NO:8) | F49Y/A51G/W93F (SEQ ID NO:11) | Wt (SEQ ID NO:1) | SEQ ID NO:38 |
| C0090 | L5Q/W97Y (SEQ ID NO:8) | F49Y/A51G/M30L/ W93F/P80S (SEQ ID NO:13) | Wt (SEQ ID NO:1) | SEQ ID NO:38 |

To evaluate hydrophobicity and aggregation the newly designed antibodies were analyzed by gel filtration (SEC) using two different SEC columns and running buffer conditions and by hydrophobic interaction chromatography (HIC) and compared to anti-oxMIF antibody C0008.

For SEC, samples were diluted to 0.1 mg/ml in 5x phosphate buffered saline including 0.02% Tween (5xPBST) and 50 µl sample was applied to a Superdex 200 increase 10/300 GL (GE Healthcare) gel-filtration column at a flow rate of 0.75 ml/min. Separations and equilibration was performed in 5xPBST at 22°C. Protein peaks were monitored using absorbance at 214 nm and spectra were analyzed using the Unicorn emulation software package (GE Healthcare). Additionally, samples were diluted to 0.2 mg/ml in 1x phosphate buffered saline (1xPBS) and 100 µl sample was applied to an Enrich 650 (Bio-Rad) gel-filtration column at a flow rate of 1 ml/min. Separations and equilibration was performed in 1xPBS at 22°C. Protein peaks were monitored using absorbance at 280 nm and spectra were analyzed using the ChromLab software package (Bio-Rad). Results are reported in retention volume (Vr, ml) for the main peak and presence of aggregates was ranked manually.

For HIC analysis, all samples were diluted to a final concentration of 0.2 mg/ml using 50mM phosphate and 0.75 M Ammonium Sulphate, pH 6.9. Highly purified samples of antibodies were loaded independently onto a 1ml HiTrap Butyl HP column. 50 µl samples were injected and the column flow rate was maintained at 1 ml/min at 22°C. Separation of peaks was carried out over a 20-column volume (CV) gradient from 0-100%B (Buffer B: 50 mM phosphate, 20% isopropanol; pH7.0). Protein peaks were monitored using absorbance at 280 nm and spectra were analyzed using the Unicorn emulation software package (GE healthcare). Results are reported in Vr (ml) at peak maximum, for each peak.

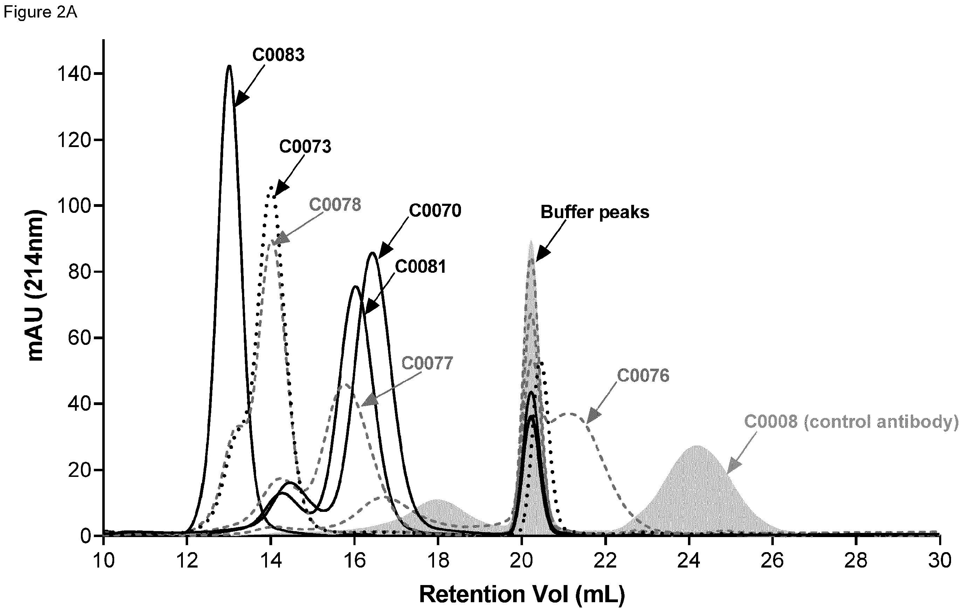

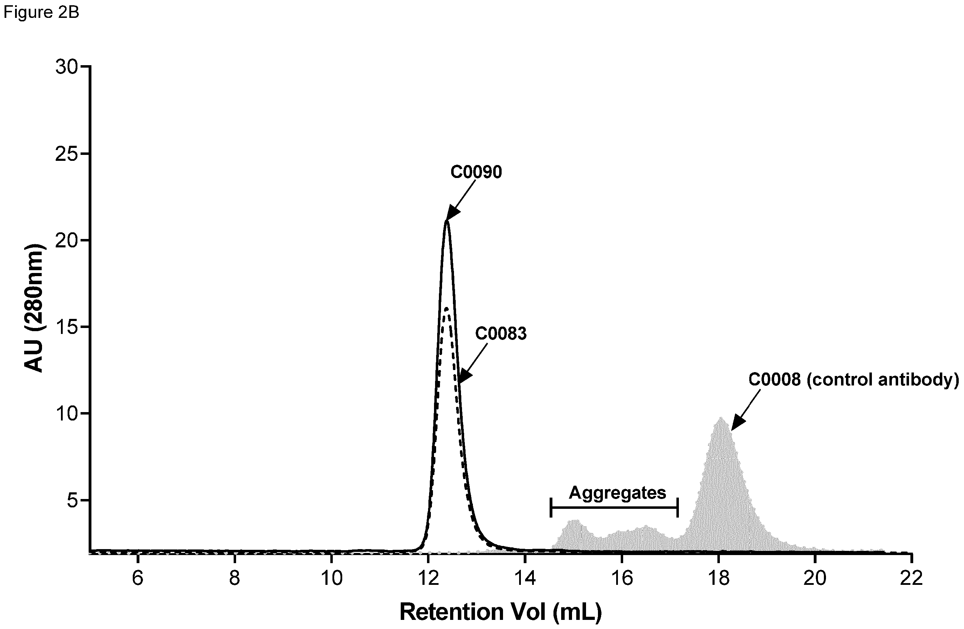

Results: Control antibody (C0008) demonstrated a retention volume close to the bed volume of the size exclusion columns (~24 ml Superdex 200, ~18 ml Enrich 650), which corresponds to a molecular weight far smaller than expected for a human IgG (Figure 2A and B). The unusual long retention was mainly due to hydrophobic interactions with the stationary phase surface. In addition, to the high retention volume, significant amounts of IgG dimers and aggregates were present for C0008. All newly designed antibodies showed a reduced retention volume (Vr) demonstrating reduced interaction with the column and thus reduced hydrophobicity of the molecules (Table 2, Figure 2A and B). C0083 and C0090 showed the lowest retention volume (Table 2, Figure 2B) and the Vr corresponded to the molecular weight of a monomeric human IgG, when compared to a molecular weight standard. Additionally, antibody dimers and aggregation were significantly reduced in samples of newly designed antibodies C0073, C0078, C0083 and C0090 (Figure 2 A and B). Especially C0083 and C0090 showed a single monomer peak.

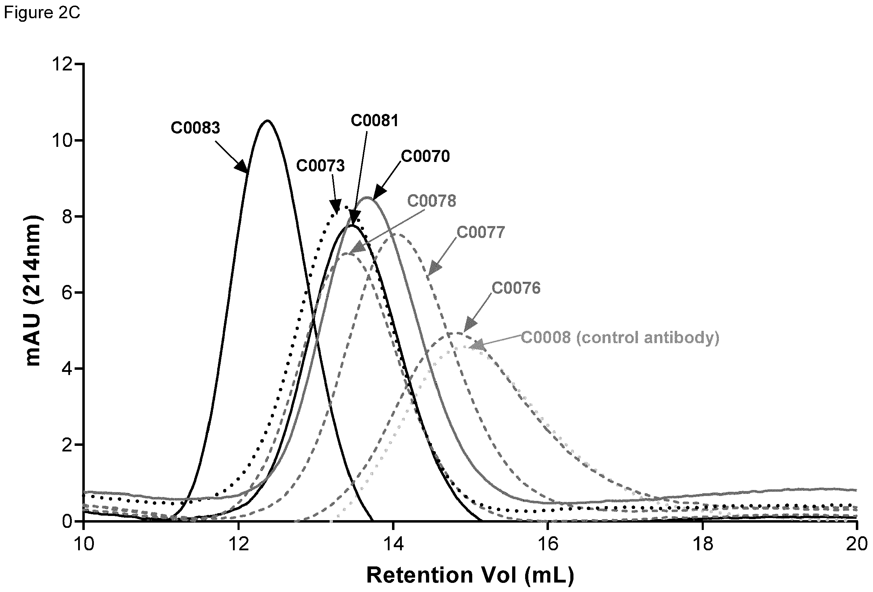

Using the HIC column retention volumes, antibodies were ranked from the least to the most (lowest to highest retention volume) hydrophobic IgG (Figure 2C, Table 3), and the results confirmed the data from SEC analysis. The newly designed antibody C0083 (retention volume of 12.37) was the least hydrophobic, whereas C0008 (retention volume of 14.92 ml) showed the highest hydrophobicity.

**Table 2 Size Exclusion Chromatography**

| **Sample** | **Running Buffer** | **Column** | **Vr (ml)** | **Aggregates** |
|---|---|---|---|---|
| C0008 | 5xPBS | Superdex 200 | 24.20 | ++ |
| C0070 | 5xPBS | Superdex 200 | 16.43 | + |
| C0073 | 5xPBS | Superdex 200 | 14.00 | (+) |
| C0076 | 5xPBS | Superdex 200 | 21.10 | + |
| C0077 | 5xPBS | Superdex 200 | 15.77 | + |
| C0078 | 5xPBS | Superdex 200 | 14.02 | (+) |
| C0081 | 5xPBS | Superdex 200 | 16.03 | + |
| C0083 | 5xPBS | Superdex 200 | 13.01 | - |
| C0008 | 1xPBS | Enrich 650 | 18.06 | + |
| C0083 | 1xPBS | Enrich 650 | 12.39 | - |
| C0090 | 1xPBS | Enrich 650 | 12.38 | - |

**Table 3 Hydrophobic Interaction Chromatography**

| **Sample** | **Vr (ml)** |
|---|---|
| C0008 | 14.92 |
| C0070 | 13.67 |
| C0073 | 13.37 |
| C0076 | 14.84 |
| C0077 | 14.05 |
| C0078 | 13.41 |
| C0081 | 13.48 |
| C0083 | 12.37 |

Figure 2 shows the chromatography profiles demonstrating reduced aggregation and hydrophobicity of the newly designed anti-oxMIF antibodies. (A) Comparison of elution profiles of C0008 (control antibody, gray area) and newly designed antibodies on a Superdex 200 increase 10/300 GL gel filtration column using 5xPBST as mobile phase. (B) Comparison of elution profiles of C0008 (control antibody, gray area) and newly designed antibodies on a Enrich 650 gel filtration column using 1xPBS as mobile phase (C) Comparison of elution profiles of C0008 (control antibody, gray area) and newly designed antibodies on a HiTrap Butyl HP HIC column.

### Example 2 Binding of newly designed anti-oxMIF antibodies to immobilized MIF (K_{D} determination)

Recombinant human MIF diluted in PBS at 1 µg/ml was immobilized into ELISA plates overnight at 4°C (transforming MIF to oxMIF according to Thiele et al., 2015). After blocking, serial dilutions of anti-oxMIF antibodies were added to the plates. Finally, bound antibodies were detected using a protein L-HRP conjugate and tetramethylbenzidine (TMB) as substrate. The chromogenic reaction was stopped with 3M H₂SO₄ and OD was measured at 450 nm. Data from different experiments were normalized to the maximal OD of the anti-oxMIF antibody C0008 (=100%) of the respective experiment and EC₅₀ values were determined by 4-parameter fit using GraphPad Prism (and the mean of two experiments is shown).

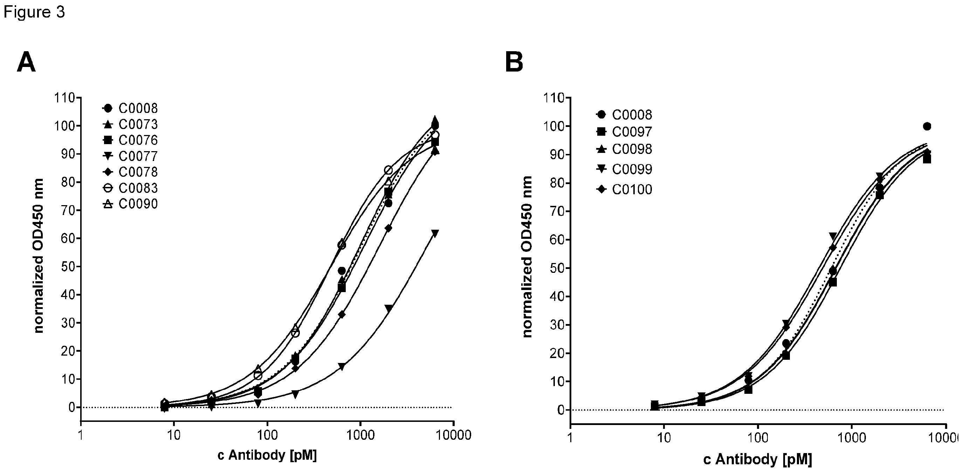

Results: The binding of the newly designed antibodies towards immobilized MIF (oxMIF) was measured over a broad range of concentrations and the resulting binding curves are illustrated in Figure 3. The anti-oxMIF antibody (C0008) was used as reference for oxMIF binding. The binding curves and the calculated EC₅₀ values, representing the K_{D}, clearly showed that the newly designed antibodies C0073, C0076, C0078, C0083 and C0090 retained their affinity (EC50 range 0.5-1.6 nM, which is within the assay variability) to oxMIF, compared to C0008 (EC50 0.7 nM) (Figure 3A). Surprisingly, C0077 showed a 5-fold reduction in affinity to oxMIF by only one additional point mutation (Y36F) in VL compared to C0076 (Figure 3A), although hydrophobicity of C0077 was markedly reduced (Figure 2A).

In another set of experiments, newly designed antibodies harboring mutations in the Fc portion to increase effector functions, showed EC₅₀ values in the range of 0.4-0.8 nM, which again exactly matches the apparent affinity of the control antibody C0008 (Figure 3B).
Figure 3 shows the binding of newly designed anti-oxMIF antibodies to immobilized MIF (K_{D} determination). (A) Binding curves of newly designed antibodies with wild-type Fc-portions. (B) Binding curves of newly designed antibodies harboring mutations in the Fc portion to increase effector functions. C0008 was used as reference antibody.

**Table 4 K_{D} values of anti-oxMIF antibodies**

| **Sample** | **K_{D} (nM)** |
|---|---|
| C0008 | 0.7 |
| C0073 | 1.0 |
| C0076 | 1.1 |
| C0077 | 5.2 |
| C0078 | 1.6 |
| C0083 | 0.5 |
| C0090 | 0.5 |
| C0097 | 0.8 |
| C0098 | 0.7 |
| C0099 | 0.4 |
| C0100 | 0.5 |

### Example 3: Differential binding of newly designed anti-oxMIF antibodies to oxMIF vs. redMIF.

Anti-oxMIF antibodies were immobilized into microplates over night at 4°C at a concentration of 15 nM. After blocking, wells were incubated with 50 ng/ml of either redMIF or the oxMIF surrogate NTB-MIF (Schinagl et al., 2018). Captured oxMIF was detected with a biotinylated polyclonal rabbit anti-MIF antibody and Streptavidin-HRP conjugate. Plates were stained with tetramethylbenzidine (TMB) and chromogenic reaction was stopped by addition of 30% H₂SO₄. OD was measured at 450 nm. Data from different experiments were normalized to the maximal OD of the anti-oxMIF antibody C0008 (=100%) of the respective experiment, and the mean of two to three experiments is shown.

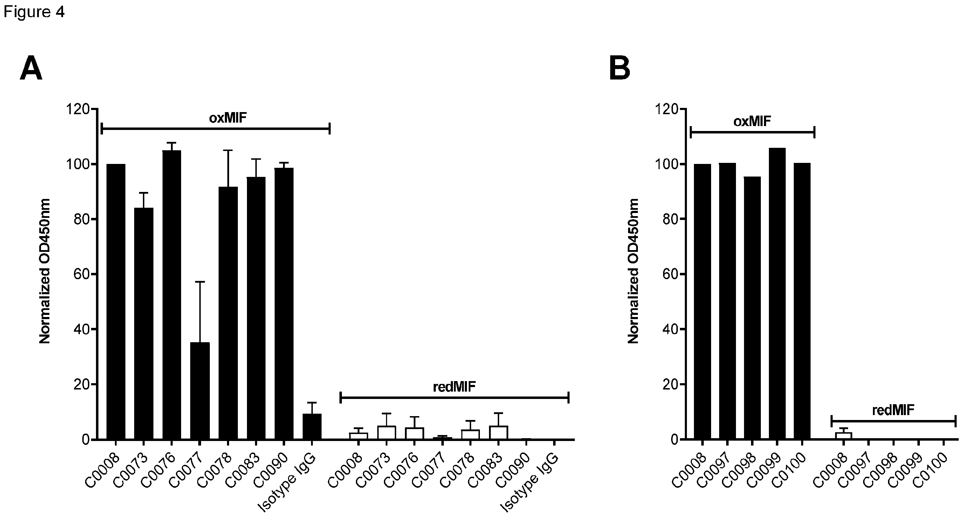

Results: The binding of the newly designed antibodies towards soluble oxMIF and redMIF are depicted in Figure 4. The results clearly showed that the newly designed antibodies C0073, C0076, C0078, C0083 and C0090 demonstrated strong binding to oxMIF but no binding to redMIF, thus the antibodies with reduced hydrophobicity and aggregation retained their ability to discriminate between oxMIF and redMIF. The newly designed antibodies showed similar ODs compared to the control antibody C0008, which has been described to discriminate between oxMIF and redMIF (Thiele et al., 2015) (Figure 4A). Surprisingly, C0077 showed that binding to oxMIF was significantly reduced by only one additional point mutation (Y36F) in VL compared to C0076 (Figure 4A). C0077 shows markedly reduced hydrophobicity (Figure 2A) but failed to maintain the binding properties.

In another set of experiments, newly designed antibodies containing mutations in the Fc portion (C0097-C0100) to increase effector functions, also retained their ability to discriminate between oxMIF and redMIF, and showed similar ODs compared to the antibody C0008 (Figure 4B).

Figure 4 shows the differential binding of the newly designed antibodies to oxMIF vs. redMIF. (A) Differential binding of newly designed antibodies with wild-type Fc-portions. (B) Differential binding of newly designed antibodies harboring mutations in the Fc portion to increase effector functions. C0008 was used as reference antibody.

### Example 4: Enhanced effector functions by Fc-mutated newly designed anti-oxMIF antibodies.

As described above, efforts can be made to increase ADCC potential of antibodies by mutations in the Fc portion. Fc receptor-mediated antibody-dependent cell-mediated cytotoxicity (ADCC) is an important mechanism of action by which antibodies target diseased cells for elimination. When the Fc effector portion of target-bound antibodies also binds to FcyRIIIA receptors on the cell surface of effector cells, multiple cross-linking of the two cell types occurs, leading to pathway activation of ADCC. Engineered Jurkat cells stably expressing the FcyRIIIA receptor, V158 (high affinity) variant, and an NFAT response element driving expression of firefly luciferase as effector cells, were used to determine the ADCC activity of the antibodies, which was quantified through the luciferase produced as a result of NFAT pathway activation.

The ADCC reporter assay was essentially performed as recommended by the manufacturer (Promega #G7010) with some modifications. Briefly, recombinant human MIF diluted in PBS at 1 µg/ml was immobilized into ELISA plates overnight at 4°C (transforming MIF to oxMIF according to Thiele et al., 2015). After blocking, serial dilutions of the newly designed antibodies described herein (C0097-C0100) were added together with Jurkat effector cells and the plates were incubated for 6h at 37°C / 5%CO₂ in a humidified incubator. Finally, Bio-Glo Luciferase reagent was added and luminescence (RLU) was measured on a Tecan multiplate reader (0.5 s integration time). C0008 was used as reference antibody.

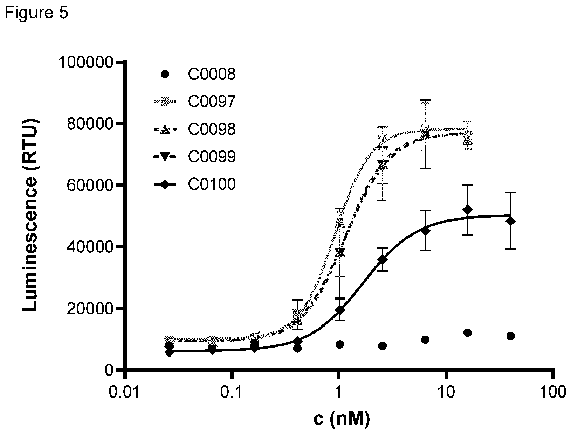

Results: All newly designed variants harboring Fc mutations (C0097-C0100) showed a significant dose response with increasing concentrations of the newly designed antibodies, whereas the antibody C0008 did not induce FcyRIIIA mediated activation of Jurkat reporter cells (Figure 5).

Figure 5 shows enhanced effector functions of newly designed anti-oxMIF antibodies in a modified ADCC Reporter Bioassay (Promega #G7010). In contrast to C0008 all newly designed anti-oxMIF antibodies induce FcyRIIIA mediated activation of Jurkat reporter cells (n=2).

### Example 5: Biodistribution of newly designed anti-oxMIF antibodies

Biodistribution of newly designed anti-oxMIF antibody C0083 in comparison to C0008 was investigated in female Balb/c mice carrying subcutaneous tumors of the syngeneic colon cancer model CT26. Female Balb/c mice received unilateral, subcutaneous injections of 3×10⁵ CT26 cells in PBS (100 µl/animal). Upon reaching individual tumor volumes of 150-300 mm³, mice were assigned to treatment groups and received a single intravenous dose of 5mg/kg/d IRDye 800CW-labeled C0008 or IRDye 800CW labeled C0083. Two mice were used as untreated 'no signal' controls.

C0008 and C0083 were labelled with IRDye 800CW using the IRDye 800CW Protein labeling kit - high MW from LI-COR Biosciences following the manufacturer's instructions. After the labelling process and prior to injection of labeled antibodies into mice, the protein concentration and labeling efficiency of the IRDye 800CW labeled antibody was determined using the Nanodrop technology, and mice were dosed based on the protein concentration after labelling. In vivo imaging was performed in a LI-COR Pearl^{®} Trilogy imaging device upon administration of labelled antibodies at the following time-points: 1h, 6-8h, 24h, 48h, 72h, 96h, 168h after dosing. Image analysis was performed to quantify the relative fluorescence units (RFU) of the antibody in the tumor (RFU= RFU tumor area - RFU background)

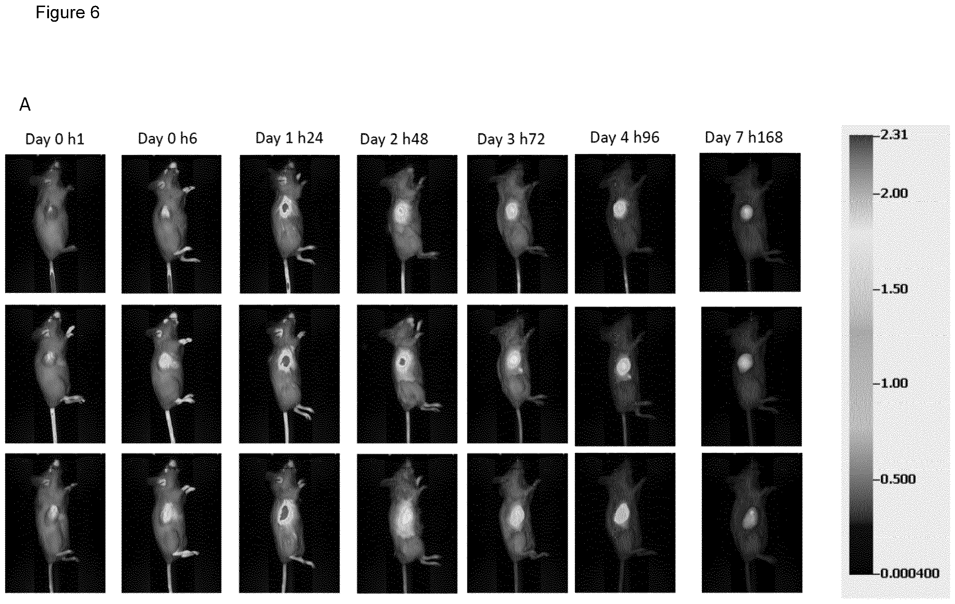

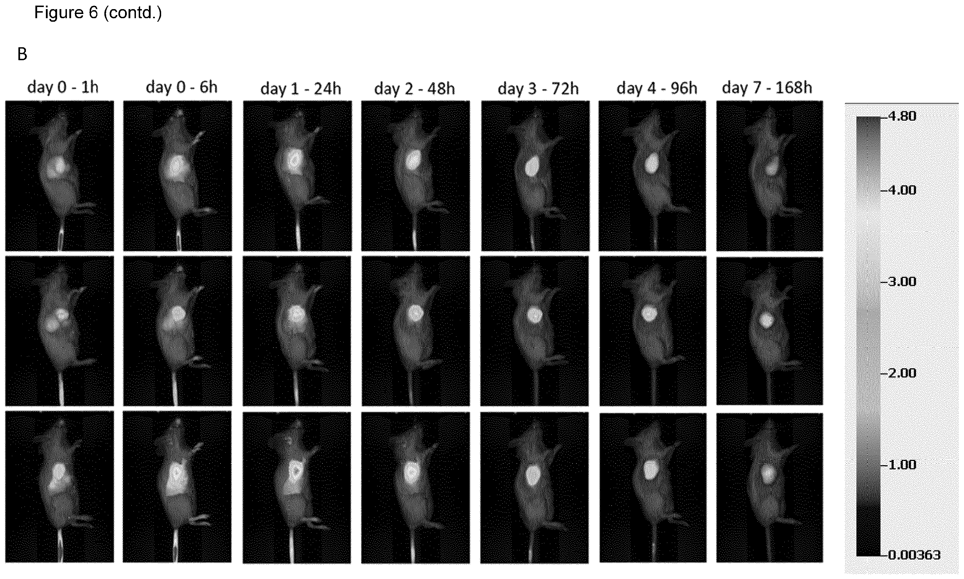

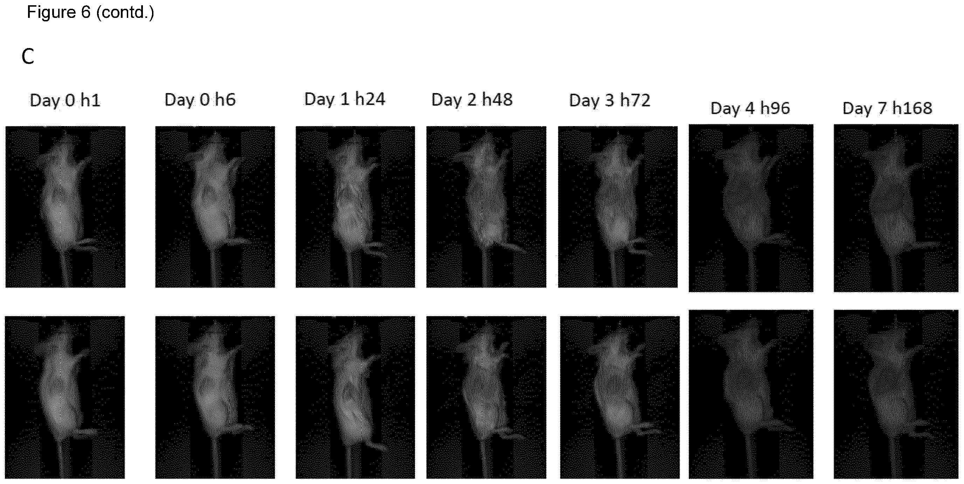

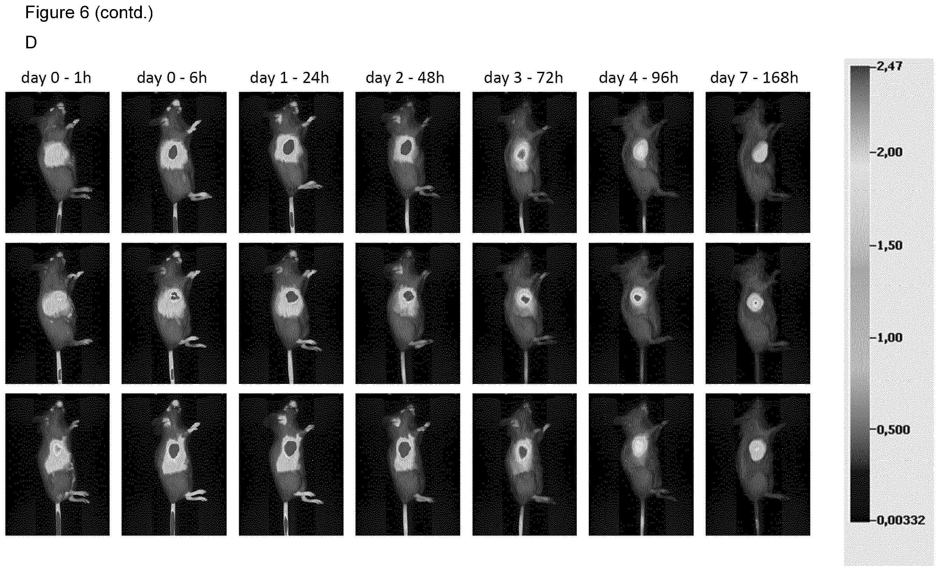

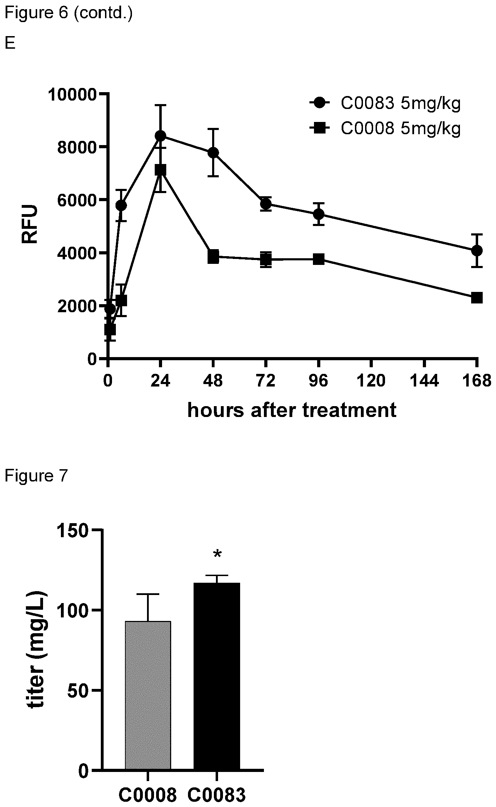

Results: A significant intra-tumoral distribution of intravenously administered 800CW-labeled C0008 (Figure 6A) and C0083 (Figure 6B) with a peak at 24h and tumor retention up to 7 days was determined. This clearly demonstrates that tumor penetration properties of C0083 were retained or even improved compared to the control anti-oxMIF antibody C0008. The two antibodies were tested in individual experiments and the scaling of the images was optimized for the respective experiment. However, using the same scaling for both experiments, the improved tumor penetration and retention of C0083 (Figure 6D) compared to C0008 becomes much more apparent. If the pixel intensity in the tumor region is evaluated digitally, the improved tumor penetration and retention of C0083 is again much more obvious (Figure 6E). No signal was detected in untreated control mice (Figure 6C).

Figure 6 shows the tumor penetration of newly designed anti-oxMIF antibody C0083 by infra-red in vivo imaging of mice carrying subcutaneous CT26 tumors. Pictures were taken 1h, 6h, 24h, 48h, 72h, 96h and 168h post injection of the IRDye 800CW labeled antibody. A: Mice which received IRDye 800CW-labeled C0008 (5mg/kg); B: Mice which received IRDye 800CW-labeled C0083 (5mg/kg/d); C: non-treated control mice, D: Mice which received IRDye 800CW-labeled C0083 (5mg/kg/d) and images are illustrated using the same scaling as in A; E: tumor penetration and retention of C0083 and C0008 quantified by digital image analysis

### Example 6: Improved production of newly designed antibody C0083.

The newly designed anti-oxMIF antibody C0083 was produced by transient expression in ExpiCHO cells (Thermo Fisher) in comparison to C0008. Briefly, 50-200 ml of exponentially growing ExpiCHO cells were transiently transfected using ExpiFectamine CHO Kit (Thermo Scientific) and were cultured for 8 days according to the manufacturer's instructions "standard protocol" (Thermo Scientific) at 37°C in a humidified incubator. Cells were removed by centrifugation and the supernatants were diluted 1:1 with 40 mM sodium phosphate, 300 mM sodium chloride, pH 7.2. Diluted supernatants were 0.2 µm filtered and were applied to a Mab Select Prism A column (Cytiva) using an NGC QUEST 10 Chromatography System (Bio-Rad) at room temperature. The column was washed with 10 column volumes of 20 mM sodium phosphate, 150 mM sodium chloride, pH 7.2 and antibodies were eluted in 10 column volumes of 100 mM glycine, pH 3.5. Antibodies were immediately neutralized to pH 5 and formulated in 1xPBS pH 7.2 by Bio-Scale P-6 desalting cartridges and were concentrated to ~1mg/ml using Amicon Ultra-15 centrifugal devices (Merck Millipore). Protein concentration was determined using NanoDrop technology and their respective extinction coefficients. The amount of purified antibody after Mab Select Prism A and neutralization (final yield of the respective antibody) was divided by the cell culture volume and the resultant value is herein referred to as antibody titer (mg/L).

Results: 3 individual productions of C0083 in comparison to C0008 clearly showed that the newly designed antibody C0083 was produced significantly (unpaired t-test, p=0.03) better. Higher antibody titers (117 mg/L) compared to C0008 (93 mg/L) were obtained (Figure 7).

Figure 7 shows the improved production of newly designed anti-oxMIF antibody C0083.

### Example 7: Improved production of newly designed anti-oxMIF antibodies with enhanced ADCC activity.

The anti-oxMIF antibodies (C0097, C0098 and C0100) harboring mutations to reduce hydrophobicity and aggregation propensity, but also Fc-mutations to improve ADCC activity were produced by transient expression in ExpiCHO cells (Thermo Fisher). Briefly, 50 ml of exponentially growing ExpiCHO cells were transiently transfected using ExpiFectamine CHO Kit (Thermo Scientific) and were cultured for 8 days according to the manufacturer's instructions ("standard protocol", Thermo Scientific) at 37°C in a humidified incubator. Cells were removed by centrifugation and 1/10 volume of 200 mM sodium phosphate, 1500 mM sodium chloride, pH 7.2 was added to the supernatants. Diluted supernatants were 0.2 µm filtered and were applied to a HiTrap Fibro PrismA column (Cytiva) using an NGC QUEST 10 Chromatography System (Bio-Rad) at room temperature. The column was washed with 10 column volumes of 20 mM sodium phosphate, 150 mM sodium chloride, pH 7.2 and antibodies were eluted in 10 column volumes of 100 mM glycine, pH 3.5. Antibodies were immediately neutralized to pH 5 with glycine to a final concentration of 250 mM glycine. Protein concentration was determined using NanoDrop technology and their respective extinction coefficients. The amount of purified antibody after HiTrap Fibro Prism A and neutralization (final yield of the respective antibody) was divided by the cell culture volume and the resultant value is herein referred to as antibody titer (mg/L). For comparison, anti-oxMIF antibodies without VH/VL optimization regarding hydrophobicity/aggregation, but harboring the identical Fc-mutations to improve ADCC activity (C0064, C0065 and C0067), were produced by the same procedure.

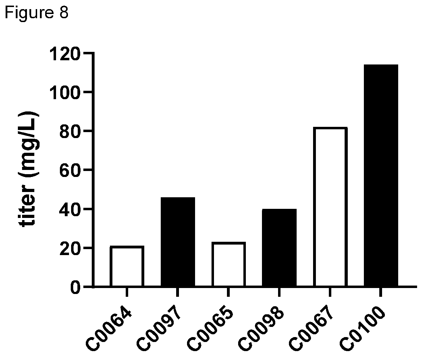

Results: Production of anti-oxMIF antibodies with improved hydrophobicity/aggregation propensity (C0097, C0098 and C0100) resulted in significantly higher titers compared to their respective counterparts, comprising VH and VL domains of C0008 (C0064, C0065 and C0067) (Figure 8).

Figure 8 shows the improved production of the newly designed anti-oxMIF antibodies with enhanced effector functions and reduced hydrophobicity and aggregation propensity, compared to their respective counterparts with enhanced effector functions, but comprising VH and VL domains of C0008 (comparison of C0097 vs C0064, C0098 vs C0065 and C0100 vs C0067).

### Example 8: Reduced aggregation propensity and reduced hydrophobicity of newly designed anti-oxMIF antibodies with enhanced effector functions

To evaluate hydrophobicity and aggregation the newly designed antibodies were analyzed by gel filtration (SEC) and by hydrophobic interaction chromatography (HIC) compared to anti-oxMIF antibody C0008.

For SEC, samples were diluted to 1 mg/ml in 1x phosphate buffered saline (1xPBS) and 100 µl sample was applied to an Enrich 650 (Bio-Rad) gel-filtration column at a flow rate of 1.25 ml/min. Separations and equilibration were performed in 1xPBS at room temperature. Protein peaks were monitored using absorbance at 280 nm and spectra were analyzed using the ChromLab software package (Bio-Rad). Results were reported in retention volume (Vr, ml) for the main peak and presence of aggregates was ranked manually.

For HIC analysis, all samples were diluted to a final concentration of 1 mg/ml using 50 mM phosphate and 0.75 M Ammonium Sulphate, pH 6.8. Highly purified samples of antibodies (~ 100 µg) were loaded independently onto a 1ml HiTrap Butyl HP column. 100 µl samples were injected and the column flow rate was maintained at 1 ml/min at room temperature. Separation of peaks was carried out over a 20-column volume (CV) gradient from 0-100%B (Buffer B: 50 mM phosphate, 20% isopropanol; pH 7.0). Protein peaks were monitored using absorbance at 280 nm and spectra were analyzed using the Unicorn emulation software package (GE healthcare). Results were reported in Vr (ml) at peak maximum, for each peak.

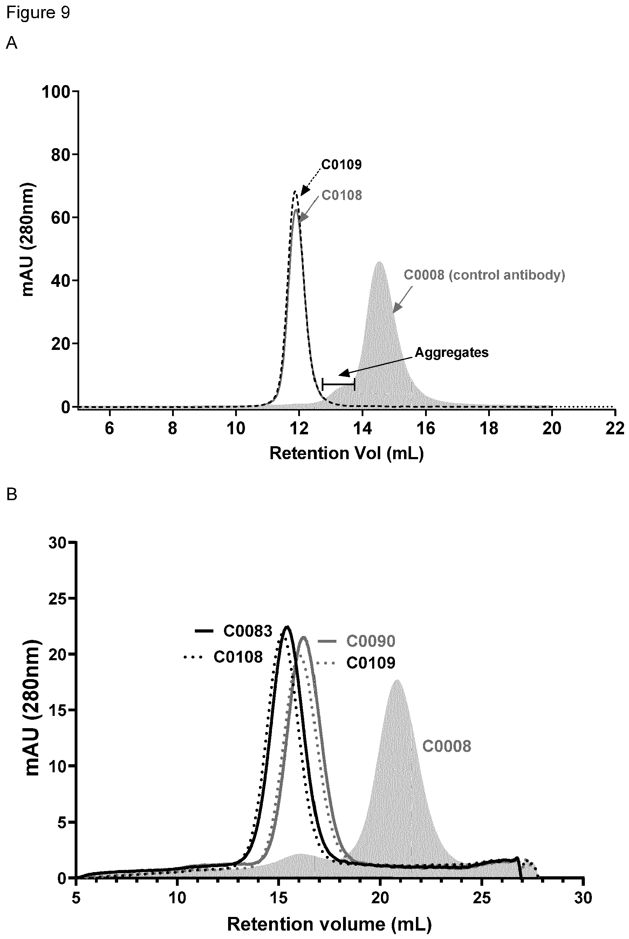

Results: Control antibody C0008 demonstrated a retention volume close to the void volume of the size exclusion column (~16 mL), which corresponds to a molecular weight far smaller than expected for a human IgG (Figure 9A). The unusual long retention was mainly due to hydrophobic interactions with the stationary phase surface. In addition, to the high retention volume, significant amounts of IgG dimers and aggregates were present for C0008. The newly designed antibodies C0108 and C0109 showed a significantly reduced retention volume (Vr) demonstrating reduced interaction with the column and thus reduced hydrophobicity of the molecules (Figure 9A, Table 5). The Vr corresponded to the molecular weight of a monomeric human IgG, when compared to a molecular weight standard. Additionally, antibody dimers and aggregation were absent in samples of newly designed antibodies C0108 and C0109 (Figure 9A).

Using HIC the Vr is directly proportional to the hydrophobicity of the analyzed antibodies, i.e. the higher the Vr the more hydrophobic is the molecule. HIC analysis of the newly designed antibodies (C0083, C0090, C0108 and C0109) confirmed that they are significantly less hydrophobic (Figure 9B, retention volume of 15.2-16.2 ml, Table 6) compared to the control antibody C0008 (Figure 9B, retention volume of 20.8 ml, Table 6).

Conclusion: It is evident from SEC and HIC analysis that the newly designed antibodies have improved biochemical properties, in particular reduced hydrophobicity, and aggregation propensity.

**Table 5 Size Exclusion Chromatography**

| *Sample* | *Running Buffer* | *Column* | *Vr (ml)* | *Aggregates* |
|---|---|---|---|---|
| C0008 | 1xPBS | Enrich 650 | 14.5 | ++ |
| C0108 | 1xPBS | Enrich 650 | 11.9 | - |
| C0109 | 1xPBS | Enrich 650 | 11.9 | - |

**Table 6 Hydrophobic Interaction Chromatography**

| *Sample* | *Vr (ml)* |
|---|---|
| C0008 | 20.8 |
| C0083 | 15.4 |
| C0090 | 16.2 |
| C0108 | 15.2 |
| C0109 | 16.0 |

Figure 9 shoes the chromatography profiles demonstrating reduced aggregation and hydrophobicity of the newly designed anti-oxMIF antibodies. **(A)** Comparison of elution profiles of C0008 (control antibody, gray area) and newly designed antibodies C0108 and C0109 with enhanced effector functions on a Enrich 650 gel filtration column using 1xPBS as mobile phase; **(B)** Comparison of elution profiles of C0008 (control antibody, gray area) and newly designed antibodies C0083, C0090, C0108 and C0109 on a HiTrap Butyl HP HIC column.

### Example 9: Reduced unspecific binding of the newly designed anti-oxMIF antibodies C0083 and C0090 to A2780 MIF^{-/-} cells.

A2780 MIF ^{-/-} cell line was generated by CRISPR/Cas9 gene editing of human MIF gene in A2780 ovarian carcinoma cell line. Absence of endogenous human MIF protein in the A2780 MIF^{-/-} cell line was confirmed by Sanger sequencing and by Western blotting using polyclonal anti-human MIF antibodies.

A2780 MIF ^{-/-} cells were detached with Cell Stripper (Corning, Cat# 25-056-CI), washed with staining buffer (PBS +5% BSA) and plated into 96-well U-bottom plate at 2×10⁵ cells per well. Cells were stained with fixable viability dye eFluo780 (diluted 1:2000 in PBS) for 20 min at 4°C and washed with staining buffer. Cells were resuspended in 50 µl of staining buffer, and 50 µl of serial dilutions of the newly designed anti-oxMIF antibodies C0083, C0090, C0108, C0109, the control anti-oxMIF antibody C0008 or an isotype IgG (final concentrations 37 nM -9.4 nM) were added. After incubating for 40 min at 4°C, cells were washed with staining buffer, and resuspended in 100 µl of secondary antibody (goat anti-human IgG (H+L)-AlexaFluor 488, diluted 1:100). After incubating for 30 min at 4°C, cells were washed with staining buffer, resuspended in PBS+2% BSA and acquired on the Cytoflex-S flow cytometer. Data were analyzed with FlowJo and the GeoMean (mean fluoresence intensity for AF488), of viable cells was plotted against antibody concentration in GraphPad Prism.

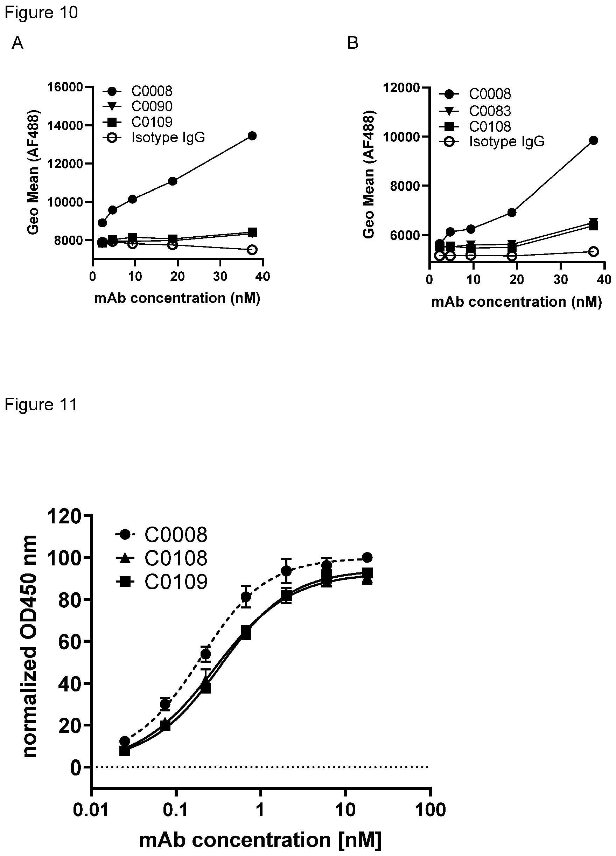

Results and Conclusion: It is apparent from Figure 10 that the newly designed anti-oxMIF antibodies C0083, C0090, C0108 and C0109 do not bind to A2780 MIF^{-/-}cells, as their Geo Mean is very close to those of the isotype IgG negative control. In contrast, the reference anti-oxMIF antibody C0008 showed significant binding to A2780 MIF^{-/-} cells, although they do not express MIF. Thus, reduction of hydrophobicity resulted in a strong reduction or elimination of unspecific binding of the newly designed anti-oxMIF antibodies C0083, C0090, C0108 and C0109 to A2780 MIF^{-/-}, whereas the anti-oxMIF control antibody C0008 binds unspecifically to the cell surface due to its hydrophobic properties.

Figure 10 shows reduced unspecific binding of the newly designed anti-oxMIF antibodies on A2780 MIF^{-/-} cells determined by FACS. A2780 MIF^{-/-} cells were stained with the newly designed anti-oxMIF antibodies C0090, C0109 **(A);** C0083, C0108 **(B)** or the control antibody C0008 as well as an isotype IgG as negative control. GeoMean (mean fluorescence intensity for AF488), of viable cells was plotted against antibody concentration.

### Example 10: Binding of newly designed anti-oxMIF antibodies with enhanced effector functions to immobilized MIF (K_{D} estimation)

Recombinant human MIF diluted in PBS at 1 µg/ml was immobilized into ELISA plates overnight at 4°C (transforming MIF to oxMIF according to Thiele et al., 2015). After blocking, serial dilutions of anti-oxMIF antibodies were added to the plates. Finally, bound antibodies were detected with Goat anti-human IgG(Fc)-HRP conjugate and tetramethylbenzidine (TMB) as substrate. The chromogenic reaction was stopped with 3M H₂SO₄ and OD was measured at 450 nm. Data from different experiments were normalized to the maximal OD of the anti-oxMIF antibody C0008 (=100%) of the respective experiment and EC₅₀ values were determined by 4-parameter fit using GraphPad Prism (mean +/- SEM of two experiments are shown).

Results and conclusion: The binding of the newly designed antibodies with enhanced effector functions towards immobilized MIF (oxMIF) was measured over a broad range of concentrations and the resulting binding curves are illustrated in Figure 11. The anti-oxMIF antibody C0008 was used as reference for oxMIF binding. The binding curves and the calculated EC₅₀ values, representing the K_{D}, clearly showed that the newly designed antibodies C0108 and C0109 retained their low nanomolar affinity to oxMIF, compared to C0008 (Figure 11, Table 7).

Figure 11 shows the binding curves of newly designed anti-oxMIF antibodies with enhanced effector functions to immobilized oxMIF (K_{D} estimation); anti-oxMIF antibody C0008 was used as reference antibody.

**Table 7: Affinity estimation of anti-oxMIF antibodies (ELISA)**

| *Sample* | *K_{D} (nM)* |
|---|---|
| C0008 | 0.2 |
| C0108 | 0.3 |
| C0109 | 0.3 |

### Example 11: Differential binding of newly designed anti-oxMIF antibodies with enhanced effector functions to oxMIF vs. redMIF.

Anti-oxMIF antibodies and an irrelevant human IgG1 control were immobilized into microplates over night at 4°C at a concentration of 15 nM. After blocking, wells were incubated with 50 ng/ml of either redMIF or the oxMIF surrogate NTB-MIF (Schinagl et al., 2018). Captured oxMIF was detected with a polyclonal rabbit anti-MIF antibody and goat anti-rabbit IgG-HRP. Plates were stained with tetramethylbenzidine (TMB) and chromogenic reaction was stopped by addition of 30% H₂SO₄. OD was measured at 450 nm. Data from two experiments were normalized to the maximal OD of the anti-oxMIF antibody C0008 (=100%) of the respective experiment, and the mean +/- SEM of two to three experiments is shown.

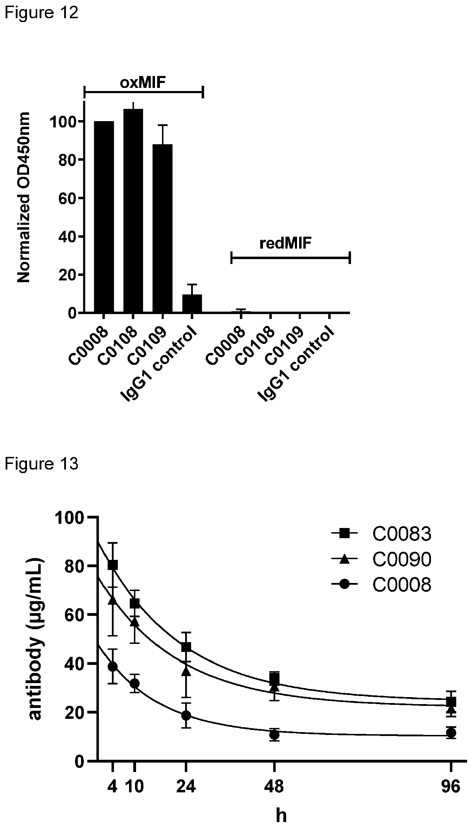

Results and conclusion: The binding of the newly designed antibodies with enhanced effector functions towards soluble oxMIF and redMIF are illustrated in Figure 12. The results clearly showed that the newly designed antibodies C0108 and C0109 demonstrated strong binding to oxMIF but no detectable binding to redMIF. The newly designed antibodies showed very comparable ODs to the reference antibody C0008, which has been described to discriminate between oxMIF and redMIF (Thiele et al., 2015). No specific binding was observed for the irrelevant IgG1 control. Thus, the newly designed antibodies with enhanced effector functions and reduced hydrophobicity and aggregation retained their ability to discriminate between oxMIF and redMIF.

Figure 12 shows differential binding of the newly designed antibodies to oxMIF vs. redMIF. C0008 was used as reference antibody and an Isotype IgG as negative control.

### Example 12: Affinity determination of newly designed antibodies towards human and mouse oxMIF using SPR

The newly designed antibodies C0108 and C0109, as well as the control anti-oxMIF antibody C0008 were immobilized to Biacore CM5 optical sensor chips (GE Healthcare) using standard amine coupling conditions to a density of about 2500, 1500 or 100 RU on Flow cells FC2, FC3 and FC4, respectively. FC1 was used as reference cell and was only reacted with buffers. Mouse or human recombinant MIF were diluted in HBS-EP buffer (GE Healthcare) to concentrations of 50, 75, 100, or 150 nM in the presence of 0.2% Proclin300 (active component 5-chloro-2-methyl-4-isothiazolin-3-one; Sigma) to transform MIF into an oxMIF surrogate (Thiele M et al., 2015) for 3 h at room temperature. Proclin300 treated MIF was applied to immobilized anti-oxMIF antibodies at a flow rate of 30 µl/min for 3 min using a Biacore ^{™} 3000 Instrument (GE Healthcare). Dissociation time was 300 seconds, and the chip was regenerated using 10mM HCl for 3 minutes. The kinetics of the concentration series were analyzed by local simultaneous association/dissociation fitting of each binding curve after subtraction of background from the reference cell (FC1) using the the iterative Langmuir 1:1 interaction model with mass transfer compensation provided by the BiaEvaluation 4.1 software (GE Healthcare). Data are represented as mean +/- SD (standard deviation) of the calculated K_{D} values of Flow Cells 2-4.

Results and conclusion: The binding of the newly designed antibodies with enhanced effector functions towards soluble human and mouse oxMIF determined by SPR are shown in Table 8. The results clearly showed that the newly designed antibodies have nanomolar affinities of about 3 nM, which is in the same range as the control anti-oxMIF antibody C0008. Affinity to mouse oxMIF was about 5-8 times lower compared to human oxMIF for all anti-oxMIF antibodies. Thus, the newly designed antibodies with enhanced effector functions and reduced hydrophobicity and aggregation retained their affinity towards human and mouse oxMIF.

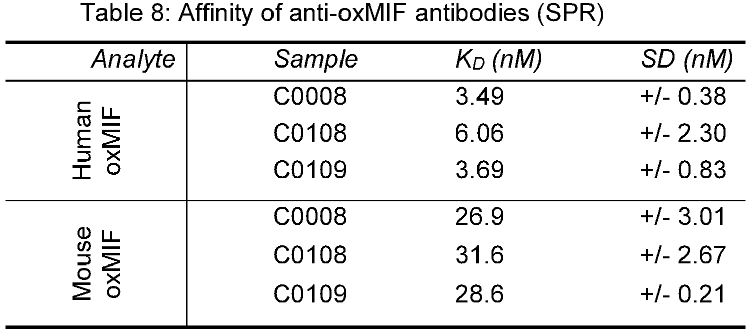

### Example 13: Pharmacokinetic of the newly designed anti-oxMIF antibodies C0083 and C0090 in comparison to control anti-oxMIF antibody C0008 in BALB/c nude mice

The newly designed anti-oxMIF antibodies C0083 and C0090 and the control anti-oxMIF antibody C0008, were injected intravenously into female BALB/c nude mice at a dose of 10 mg/kg. 20 µl of blood was collected 4, 10, 24, 48 and 96h post injection by tail vein puncture using K₂-EDTA-coated capillaries and transferred into K₂-EDTA-coated vials containing 60 µl PBS. After centrifugation, the supernatants (= 1:4-diluted plasma) were analyzed by a quantitative anti-oxMIF ELISA.

Briefly, recombinant human MIF (OncoOne) diluted in PBS at 1 µg/ml was immobilized into ELISA plates (MaxiSorp, Nunc) overnight at 4°C. After blocking with dilution buffer (DB, 2% Fish Gelatine/TBST) diluted plasma samples (final dilution 1:100-1:100,000) were added to the plates. Bound antibodies were detected using a Goat anti-Human IgG(Fc)-HRP conjugate (1:40,000 in TBST, Thermo Scientific #32420). Tetramethylbenzidine (TMB, Thermo Scientific #34029) was added as substrate and the chromogenic reaction was stopped with 50 µl 30% H₂SO₄ and OD was measured at 450 nm. Serial dilutions of C0008, C0083 and C0090 were used to generate standard curves using hyperbola regression analysis in GraphPad Prism and to calculate plasma concentration of the antibodies. The mean concentration of the antibodies per time point was plotted against collection time and half-lives were calculated by a one-phase decay model in GraphPad Prism.

Results: Figure 13 shows the pharmacokinetic of the anti-oxMIF antibodies following a mono-exponential decay function. The half-lives calculated were 11h for control anti-oxMIF antibody C0008 and 15 h for the newly designed anti-oxMIF antibodies C0083 and C0090, respectively. Additionally, the area under the curve (AUC) below the mono-exponential decay function was calculated to estimate total antibody exposure and bioavailability. AUCs were calculated to be 1600, 3784 and 3266 for C0008, C0083 and C0090, respectively.

Pharmacokinetic of the newly designed anti-oxMIF antibodies C0083 and C0090 is shown in in comparison to the control anti-oxMIF antibody C0008 in BALB/c nude mice. Plasma concentration of the antibodies was determined by a quantitative anti-oxMIF ELISA at 4, 10, 24, 48 and 96h post single intravenous injection of 10 mg/kg anti-oxMIF antibodies and the half-lives were calculated by a one-phase decay model in GraphPad Prism.

Conclusion: Substitutions in the variable regions of the newly designed anti-oxMIF antibodies C0083 and C0090 resulted in a 1.4-fold longer half-life and > 2-fold higher bioavailability (AUC) compared to the control anti-oxMIF antibody C0008.

### Example 14: Increased effector functions of the newly designed anti-oxMIF antibodies C0108 and C0109 determined by reporter assays

Using engineered Jurkat cells either stably expressing the human FcγRIIIa receptor, V158 (high affinity allotype) or F158 (low affinity allotype) to elucidate ADCC or the human FcγRIIa H131 allotype receptor to elucidate ADCP, and a NFAT response element driving expression of firefly luciferase as effector cells, antibody biological activity was quantified through the luciferase produced as a result of NFAT pathway activation.

To generate highly responsive target cells, HCT116 and A2780 cells were transfected with a huMIF-pDisplay plasmid (Invitrogen), selected with geneticin, and sorted by FACS to generate cell lines stably expressing membrane-anchored monomeric human MIF (HCT116-pMIF or A2780-pMIF), i.e. MIF is displayed as monomeric protein in which an oxMIF epitope is accessible to anti-oxMIF antibodies (Schinagl et al., Biochemistry 2018). These cell lines show increased presentation of oxMIF at the cellular surface and are therefore a sensitive tool for in vitro analysis.

The ADCC or ADCP reporter assays were essentially performed as recommended by the manufacturer (Promega #G7010 and #G9991). In brief, 1×10⁴ cells/well of HCT116-pMIF (Figure 14 A and C) orA2780-pMIF (Figure 14 B) cells in 100 µl culture medium (RPMI 1640 medium supplemented with Pen/Strept/L-Glutamin and 5 % low IgG FBS) were seeded into 96-well plates and incubated overnight at 37°C/5 % CO₂ in a humidified incubator to adhere. Thereafter, serial dilutions of the newly designed anti-oxMIF antibodies with enhanced effector functions C0108 and C0109 or the control antibody C0008 with wt Fc (final concentration 0.01-100 nM) and Jurkat effector cells (Figure 14 A-B, FcγRIIIa effector cells; Figure 14 C, FcγRIIa-effector cells) at an effector to target cell ratio of about 6:1 were incubated for 6 h at 37°C / 5'CO₂ in a humidified incubator. Finally, the assay plates were equilibrated to room temperature and Bio-Glo Luciferase reagent was added. Luminescence (RLU) was measured after 10-20 min incubation using a Tecan multiplate reader (0.5 s integration time).

Results: It is evident from Figure 14 A-C that the newly designed antibodies C0108 and C0109 harboring ADCC/ADCP enhancing mutations in the Fc portion showed strong activation of the ADCC (Figure 14 A-B) or ADCP (Figure 14 C) reporter cells, whereas the control antibody C0008 induced no or only minor FcyRIIIa (ADCC, Figure 14 A-B) or FcyRIIa (ADCP, Figure 14 C) mediated activation of Jurkat reporter cells when they were cocultured with HCT116-pMIF (Figure 14 A, C) or A2780-pMIF (Figure 14 B) cells.

Conclusion: The newly designed anti-oxMIF antibodies C0108 and C0109 harboring ADCC/ADCP enhancing mutations in the Fc portion showed highly increased ADCC and ADCP activity in reporter bioassays, compared to wt Fc bearing control antibody C0008.

Figure 14 shows enhanced effector functions of the newly designed antibodies C0108 and C0109 determined by reporter assays. **(A-B)** ADCC reporter bioassay with the newly designed antibodies C0108 and C0109 with enhanced effector functions using engineered Jurkat effector cells stably expressing FcyRIIIa (V high responder allotype, F low-responder allotype) and HCT116-pMIF **(A)** or A2780-pMIF **(B)** target cells compared to the anti-oxMIF control antibody C0008 with wt Fc; **(C)** ADCP reporter bioassay with the newly designed antibodies C0108 and C0109 with enhanced effector functions using engineered Jurkat effector cells stably expressing FcyRIIa and HCT116-pMIF target cells compared to the anti-oxMIF control antibody C0008 with wt Fc. Mean and SEM are shown (n=2-4).

### Example 15: Enhanced ADCC function of the newly designed anti-oxMIF antibodies C0108 and C0109 determined by a PBMC cell lysis bioassay.

This assay was performed to determine cell lysis resulting from the antibody-dependent cytotoxicity (ADCC) induced by the newly designed anti-oxMIF antibodies. Cytotoxicity was measured by HiBiT Detection assay (Promega) which quantifies the release of a HiBiT tagged protein from the target cells using a non-lytic detection reagent containing LgBit (LargeBiT) and furimazine (substrate). HiBiT and LgBiT spontaneously assemble to a functional NanoBiT^{®} enzyme and emits a quantifiable luminescence signal in the presence of the substrate.

To generate highly responsive reporter target cells, HCT116 cells were transfected with the HaloTag-HiBiT plasmid (Promega #CS1956B17), selected with blasticidin and sorted by FACS as cell pools. Stable HiBiT-expressing cell pools were then transfected with a huMIF-pDisplay plasmid (Invitrogen), selected with geneticin, and sorted by FACS to generate cell pools stably expressing intracellular HiBiT and membrane-anchored monomeric human oxMIF (HCT116-HiBiT-pMIF), i.e. MIF is displayed as monomeric protein in which an oxMIF epitope is accessible to anti-oxMIF antibodies (Schinagl et al., Biochemistry 2018). These cell lines show increased presentation of oxMIF at the cellular surface and are therefore a sensitive tool for in vitro analysis.

In brief, 1×10⁴ cells/well of HCT116-HiBiT-pMIF target cells in 50 µl culture medium (RPMI 1640 medium supplemented with Pen/Strept/L-Glutamin and 5% ultra-low IgG FBS) were seeded into 96-well plates and incubated overnight at 37°C/5% CO₂ in a humidified incubator to adhere. Thereafter, 50 µl serial dilutions of the newly designed anti-oxMIF antibodies C0108 and C0109 or the control anti-oxMIF antibody C0008 with wt Fc in culture medium (final concentration 0.01-100 nM) and 50 µl of human PBMCs effector cells from two individual healthy donors (4×10⁵ cells/well; effector to target cell ratio; 40:1) were added to the plates. PBMCs of the two different donors were characterized by FACS analysis to evaluate their content of NK cells, which are the relevant cell type for this assay. After adding antibodies and PBMCs, the plate was incubated over night at 37°C and 5% CO₂ in a humidified incubator. On the next day 10 µl of Nano-Glo HiBiT Extracellular Detection Reagent (Promega #N2421) was added, and luminescence signals (RLU, integration time 0.1 s) were measured on a Tecan plate reader. The assay was performed to include duplicates or triplicates of the analyzed samples and donors.

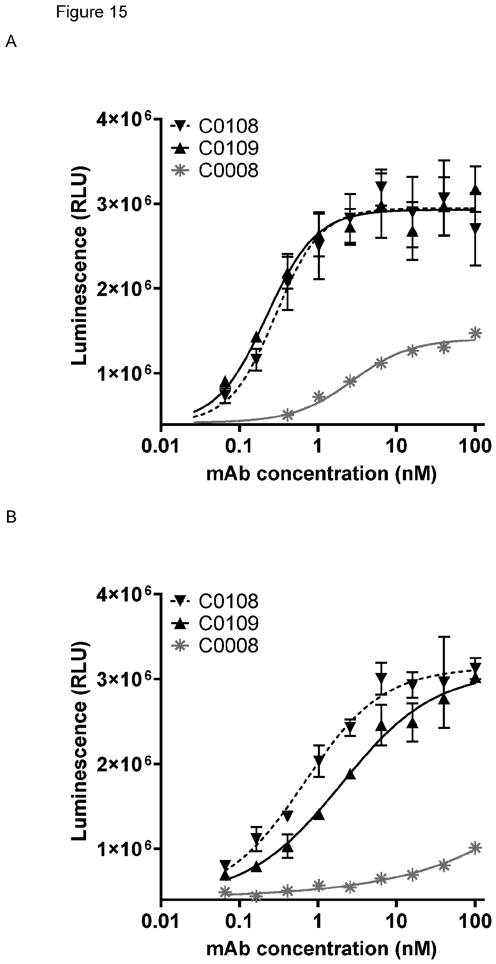

Results: It is evident from Figure 15 that the newly designed antibodies C0108 and C0109 harboring ADCC/ADCP enhancing mutations in the Fc portion showed largely increased ADCC activity with PBMCs from both donors (Figure 15A, 22% NK cells; (Figure 15B, 7% NK cells) compared to the control anti-oxMIF antibody C0008.

Conclusion: The newly designed anti-oxMIF antibodies C0108 and C0109 harboring ADCC/ADCP enhancing mutations in the Fc portion did show highly increased ADCC activity in a PBMC mediated cell killing assay, compared to wt Fc bearing control anti-oxMIF antibody.

Figure 15 shows the enhanced ADCC activity of the newly designed anti-oxMIF antibodies C0108 and C0109 harboring ADCC/ADCP enhancing mutations in the Fc portion determined by a PBMC mediated cell toxicity bioassay. ADCC bioassay with newly designed anti-oxMIF antibodies C0108 and C0109 and PBMCs as effector cells (**A:** 22% NK cells, **B:** 7% NK cells) and HCT116-pMIF as target cells compared to the anti-oxMIF control antibody C0008 with wt Fc. Mean and SEM of two or three replicates using PBMCs from two healthy donors are shown.

### Example 16: Newly designed anti-oxMIF antibodies show a strongly reduced unspecific cytokine release from human PBMCs s.

Cytokine release syndrome (CRS) is a form of systemic inflammatory response syndrome (SIRS) that can be triggered by a variety of factors such as infections. CRS is also known as an adverse effect of some monoclonal antibody medications. CRS occurs when large numbers of white blood cells, including B cells, T cells, natural killer cells, macrophages, dendritic cells, and monocytes are activated and release inflammatory cytokines, like IL-6, IFN-gamma, IL-8, MCP-1 amongst others, which activate more white blood cells in a positive feedback loop of pathogenic inflammation. This process, when dysregulated, can be life-threatening due to systemic hyper-inflammation, hypotensive shock, and multi-organ failure. Thus, the newly designed anti-oxMIF antibodies were assessed for their potential to release inflammatory cytokines from PMBCs in an in-vitro assay.

In brief, the newly designed anti-oxMIF antibodies C0108 and C0109 (both at 70 nM) and the anti-oxMIF control antibody C0008 (70 nM) were incubated with freshly thawed PBMCs (4-5×10⁵ cells per well) from three healthy donors in 150 µl of RPMI1640 medium supplemented with 5% ultra-low IgG serum in 96-well plates. After overnight incubation at 37°C/5% CO₂ in a humidified incubator, plates were centrifuged at 300 × g for 5 min to pellet the cells, and supernatants were transferred to the 96-well V-bottom plates. Cleared supernatants were analyzed by BioLegend LegendPlex cytometric bead assay for human IL-6 and human MCP-1 according to manufacturer's protocol. Measurement was done on a CytoFlex-S cytometer with 96-well plate loader (Beckman Coulter). Beads classification channel was APC (excitation from laser 638 nm, band pass filter 660/10 nm), whereas reporter channel was PE (excitation from yellow laser 561nm, band pass filter 585/42 nm). Data were analyzed using LegendPlex analysis software (BioLegend) and graphs were produced in GraphPad Prism. Mean +/- SEM are shown from 3 different PMBC donors.

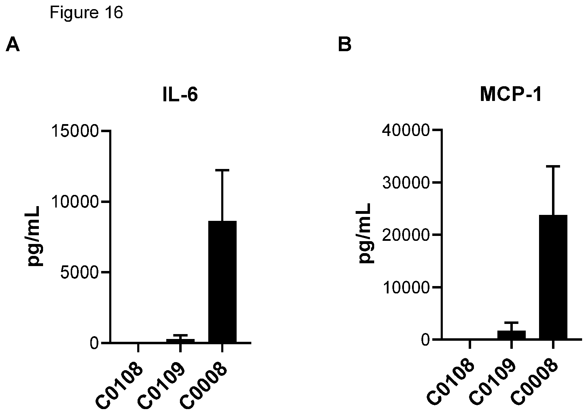

Results and Conclusion: It is evident from Figure 16 that the newly designed antibodies C0108 and C0109 harboring ADCC/ADCP enhancing mutations in the Fc portion surprisingly showed no or only negligible release of IL-6 and MCP-1 from PBMCs at concentrations up to 70 nM in comparison to C0008. It could have been expected that C0108 and C0109 induce higher levels of cytokine release due to their high affinity to Fcy-receptors than the reference anti-oxMIF antibody C0008 harboring a wt Fc-portion. Though, C0008 induced significant release of IL-6 and MCP-1 at the same concentration (70 nM) obviously due to the higher aggregation propensity and unspecific binding due to its hydrophobicity. Aggregation of antibody therapeutics, even if minor, is known to strongly potentiate cytokine release from immune cells.

Figure 16 shows that the newly designed anti-oxMIF antibodies show a strongly reduced unspecific cytokine release from human PBMCs. The newly designed anti-oxMIF antibodies C0108 and C0109 harboring ADCC/ADCP enhancing mutations in the Fc portion (70 nM) and the control anti-oxMIF antibody C0008 (70 nM) were incubated with human PBMCs overnight, and supernatants were analyzed in LegendPlex cytometric bead assays (BioLegend) for human IL-6 (A) and human MCP-1 (B). Mean +/- SEM for cytokine concentrations are shown from 3 different PMBC donors.

### REFERENCES

Bloom B.R. and Bennet, B., 1966, Mechanism of a reaction in vitro associated with delayed-type hypersensitivity, Science 153, 80-82
Brinkmann U. and Kontermann R.E., 2017, The making of bispecific antibodies, MABS, 9, 2, 182-212
David, J.R., 1966, Delayed hypersensitivity in vitro: its mediation by cell-free substances formed by lymphoid cell-antigen interaction, Proc. Natl. Acad. Sci. U.S.A. 56, 72-77
Estep P. et al., 2015, An alternative assay to hydrophobic interaction chromatography for high-throughput characterization of monoclonal antibodies, MAbs, 7(3), 553-561
Ewert S. et al., 2003, Structure-based improvement of the biophysical properties of immunoglobulin VH domains with a generalizable approach, Biochemistry, 18, 42(6), 1517-28
Hong, P., et al. (2012). A review size-exclusion chromatography for the analysis of protein biotherapeutics and their aggregates. In Journal of Liquid Chromatography and Related Technologies (Vol. 35, Issue 20, pp. 2923-2950). Taylor & Francis. https://doi. org/10.1080/10826076.2012.743724
Hussain F. et al., 2013, Human anti-macrophage migration inhibitory factor antibodies inhibit growth of human prostate cancer cells in vitro and in vivo, Mol Cancer Ther. Jul;12(7):1223-34
lanello A. and Ahmad A., 2005, Role of antibody-dependent cell-mediated cytotoxicity in the efficacy of therapeutic anti-cancer monoclonal antibodies, Cancer and Metastasis Review, 24, 487-499
Kabat et al., Sequences of Proteins of Immunological Interest, Fifth Edition, NIH Publication 91-3242, Bethesda MD (1991), vols. 1-3
Mahalingam D. et al., 2015, ASCO Abstract ID2518
Mahalingam et al. 2020, Phase I study of Imalumab (BAX69), a fully human recombinant antioxidized macrophage migration inhibitory factor antibody in advanced solid tumours, Br J Clin Pharmacol. 86(9):1836-1848
Morrison, S.L., et al., 1984, Chimeric human antibody molecules: mouse antigen-binding domains with human constant region domains, Proc. Natl. Acad. Sci. 81, 6851-6855
Natsume A. et al., 2009, "Improving effector functions of antibodies for cancer treatment: Enhancing ADCC and CDC", Drug, Design, development and Therapy, 3, 7-16
Sambrook et al, "Molecular Cloning: A Laboratory Manual" (4th Ed.), Vols. 1 -3, Cold Spring Harbor Laboratory Press (2012).
Schinagl, A., et al., 2018, Role of the Cysteine 81 Residue of Macrophage Migration Inhibitory Factor as a Molecular Redox Switch. Biochemistry, 57(9), 1523-1532. https://doi.org/10.1021/acs.biochem.7b01156
Schinagl. A. et al., 2016, Oxidized macrophage migration inhibitory factor is a potential new tissue marker and drug target in cancer, Oncotarget. Nov 8;7(45):73486-73496
Spiess C. et al., 2015, Alternative molecular formats and therapeutic applications for bispecific antibodies, Mol.Immunol., 67, 95-106
Tang J., et al., 2018, Comprehensive analysis of the clinical immuno-oncology landscape, Ann Oncol.;29(1):84-91
Thiele, M. et al., (2015). Selective Targeting of a Disease-Related Conformational Isoform of Macrophage Migration Inhibitory Factor Ameliorates Inflammatory Conditions. Journal of Immunology, 195(5), 2343-2352. https://doi.org/10.4049/jimmunol.1500572
Van der Kant, R. et al., 2017, Prediction and Reduction of the Aggregation of Monoclonal Antibodies, J.Mol.Biol., 429(8), 1244-1261
Wei Li et al., 2016, Antibody Aggregation: Insights from Sequence and Structure, Antibodies, 5(3), 19
Willuda J. et al., 1999, High thermal stability is essential for tumor targeting of antibody fragments: engineering of a humanized anti-epithelial glycoprotein-2 (epithelial cell adhesion molecule) single-chain Fv fragment, Cancer Res., 15; 59(22), 5758-67

## Claims

1. A recombinant anti-oxMIF antibody or an antigen binding fragment thereof having reduced aggregation potential and reduced hydrophobicity, comprising
the following variable domains:
(a1) a light chain variable domain comprising SEQ ID NO:9 with 1, 2, 3, 4, or 5 amino acid substitutions selected from M30L, F49Y, A51G, P80S and W93F, or
(a2) a light chain variable domain comprising SEQ ID NO:9 with 1, 2, 3, 4, or 5 amino acid substitutions selected from M30L, F49Y, A51G, P80S and W93F, and with 1, 2, 3, 4, or 5 further amino acid substitutions, with the proviso that the tyrosine at position 36 is preserved, and one of
(b1) a heavy chain variable domain comprising SEQ ID NO:6; or
(b2) a heavy chain variable domain comprising SEQ ID NO:6 with 1 or 2 amino acid substitutions selected from L5Q and W97Y, or
(b3) a heavy chain variable domain comprising SEQ ID NO:6 with 1 or 2 amino acid substitutions selected from L5Q and W97Y, and with 1, 2, 3, 4 or 5 further amino acid substitutions,
wherein amino acid positions are numbered according to Kabat, and wherein aggregation potential and hydrophobicity are reduced compared to an antibody or an antigen binding fragment thereof comprising SEQ ID NO:6 and SEQ ID NO:9 lacking the amino acid substitutions.

2. The recombinant anti-oxMIF antibody of claim 1, comprising the amino acid substitutions W93F and/or W97Y.

3. The recombinant anti-oxMIF antibody of claim 1 or 2, having enhanced effector functions, comprising a heavy chain constant region comprising
(a) SEQ ID NO:2 or a heavy chain constant region having at least 95% sequence identity to SEQ ID NO:2 and comprising amino acids 239D and 332E; or
(b) SEQ ID NO:3, or a heavy chain constant region having at least 95% sequence identity to SEQ ID NO:3 and comprising amino acids 239D, 268F, 324T and 332E; or
(c) SEQ ID NO:4, or a heavy chain constant region having at least 95% sequence identity to SEQ ID NO:4 and comprising amino acids 239D, and 332E; or
(d) SEQ ID NO:5, or a heavy chain constant region having at least 95% sequence identity to SEQ ID NO:5 and comprising amino acids 235V, 243L, 292P, 300L, and 396L,
wherein amino acid positions are numbered according to EU numbering index.

4. The anti-oxMIF antibody of any one of claims 1 to 3, comprising SEQ ID NO:8 and an amino acid sequence selected from the group consisting of SEQ ID NOs: 10, 11, 12, and 13.

5. The anti-oxMIF antibody of any one of claims 1 to 2, comprising SEQ ID NOs:6, 11 and any one of SEQ ID NOs:1, 2, 3, 4, or 5.

6. The anti-oxMIF antibody of any one of claims 1 to 2, comprising SEQ ID NOs:7, 10 and any one of SEQ ID NOs:1, 2, 3, 4, or 5.

7. The anti-oxMIF antibody of any one of claims 1 to 2, comprising SEQ ID NOs:7, 11 and any one of SEQ ID NOs:1, 2, 3, 4, or 5.

8. The anti-oxMIF antibody of any one of claims 1 to 2, comprising SEQ ID NOs:8, 11 and any one of SEQ ID NOs:1, 2, 3, 4, or 5.

9. The anti-oxMIF antibody of any one of claims 1 to 2, comprising SEQ ID NOs:8, 13 and any one of SEQ ID NOs:1, 2, 3, 4, or 5.

10. The anti-oxMIF antibody of any one of claims 1 to 2, comprising SEQ ID NOs:8, 10 and any one of SEQ ID NOs:1, 2, 3, 4, or 5.

11. The anti-oxMIF antibody of any one of claims 1 to 3, comprising SEQ ID NOs:8, 11, 4, and 38.

12. The anti-oxMIF antibody of any one of claims 1 to 3, comprising SEQ ID NOs: 8, 13, 4, and 38.

13. The anti-oxMIF antibody according to any one of claims 1 to 12, selected from the group consisting of bispecific antibodies, scFv, (scFv)2, scFvFc, Fab, Fab', and F(ab')2, Fab'-SH, Fab-scFv fusion, Fab-(scFv)2-fusion, Fab-scFv-Fc, Fab-(scFv)2-Fc, fusion proteins of two single chain antibodies of different species (BiTE), and minibody.

14. The antibody of any one of claims 1 to 13 for use in the preparation of a medicament.

15. A pharmaceutical composition comprising the antibody of any one of claims 1 to 13, optionally together with a pharmaceutical carrier or adjuvant.

16. The pharmaceutical composition according to claim 15, wherein the composition comprises 10 to 250 mg/ml of the antibody of any one of claims 1 to 13, specifically comprising > 50 mg/ml.

17. The pharmaceutical composition according to claim 15 or 16, wherein said pharmaceutical composition is formulated for subcutaneous administration.

18. The pharmaceutical composition according to any one of claims 15 to 17, wherein the composition is for administration as the sole substance, or with a further pharmaceutical composition comprising one or more active substances, preferably selected from the group consisting of antiviral, anti-inflammatory, anti-cancer, anti-angiogenic, and antibiotic substances.

19. The pharmaceutical composition according to any one of claims 15 to 18 for use in the treatment of a patient suffering from inflammatory disease, hyperproliferative disorder, infectious disease or cancer, specifically in the treatment of asthma, vasculitis, arthritis, sepsis, septic shock, endotoxic shock, toxic shock syndrome, acquired respiratory distress syndrome, glomerulonephritis, inflammatory bowel disease, Crohn's disease, ulcerative colitis, peritonitis, nephritis and psoriasis, colorectal cancer, ovarian cancer, breast cancer, prostate cancer, pancreas cancer, and lung cancer.

20. An isolated nucleic acid encoding the antibody of any one of claims 1 to 13.

21. An expression vector comprising the nucleic acid molecule(s) of claim 20.

22. A host cell containing the nucleic acid of claim 20 or an expression vector of claim 21.

23. A method of producing the antibody of any one of claims 1 to 13, comprising culturing a host cell of claim 22 and recovering said antibody from the cell culture.

## Patentansprüche

1. Rekombinanter Anti-oxMIF-Antikörper oder antigenbindendes Fragment davon, mit verringertem Aggregationspotential und verringerter Hydrophobizität, umfassend die folgenden variablen Domänen:
(a1) eine variable Domäne der leichten Kette umfassend SEQ ID NO: 9 mit 1, 2, 3, 4 oder 5 Aminosäure-Substitutionen ausgewählt aus M30L, F49Y, A51G, P80S und W93F, oder
(a2) eine variable Domäne der leichten Kette umfassend SEQ ID NO: 9 mit 1, 2, 3, 4 oder 5 Aminosäure-Substitutionen ausgewählt aus M30L, F49Y, A51G, P80S und W93F, und mit 1, 2, 3, 4 oder 5 weiteren Aminosäure-Substitutionen, mit der Maßgabe, dass das Tyrosin an Position 36 erhalten wird, und eine von
(b1) einer variablen Domäne der schweren Kette umfassend SEQ ID NO: 6; oder
(b2) einer variablen Domäne der schweren Kette umfassend SEQ ID NO: 6 mit 1 oder 2 Aminosäure-Substitutionen ausgewählt aus L5Q und W97Y, oder
(b3) einer variablen Domäne der schweren Kette umfassend SEQ ID NO: 6 mit 1 oder 2 Aminosäure-Substitutionen ausgewählt aus L5Q und W97Y, und mit 1, 2, 3, 4 oder 5 weiteren Aminosäure-Substitutionen,
wobei die Aminosäure-Positionen nach Kabat nummeriert sind, und wobei das Aggregationspotential und die Hydrophobizität im Vergleich zu einem Antikörper oder einem antigenbindenden Fragment davon, der/das SEQ ID NO: 6 und SEQ ID NO: 9 ohne die Aminosäure-Substitutionen umfasst, verringert sind.

2. Rekombinanter Anti-oxMIF-Antikörper nach Anspruch 1, umfassend die Aminosäure-Substitutionen W93F und/oder W97Y.

3. Rekombinanter Anti-oxMIF-Antikörper nach Anspruch 1 oder 2, der verbesserte Effektor-Funktionen aufweist, und eine konstante Region der schweren Kette umfasst, die umfasst
(a) SEQ ID NO: 2 oder eine konstante Region der schweren Kette mit zumindest 95 % Sequenzidentität zu SEQ ID NO: 2 und umfassend die Aminosäuren 239D und 332E; oder
(b) SEQ ID NO: 3 oder eine konstante Region der schweren Kette mit zumindest 95 % Sequenzidentität zu SEQ ID NO: 3 und umfassend die Aminosäuren 239D, 268F, 324T und 332E; oder
(c) SEQ ID NO: 4 oder eine konstante Region der schweren Kette mit zumindest 95 % Sequenzidentität zu SEQ ID NO: 4 und umfassend die Aminosäuren 239D und 332E; oder
(d) SEQ ID NO: 5 oder eine konstante Region der schweren Kette mit zumindest 95 % Sequenzidentität zu SEQ ID NO: 5 und umfassend die Aminosäuren 235V, 243L, 292P, 300L und 396L,
wobei die Aminosäure-Positionen nach dem EU-Nummerierungs-Index nummeriert sind.

4. Anti-oxMIF-Antikörper nach einem der Ansprüche 1 bis 3, umfassend die SEQ ID NO: 8 und eine Aminosäuresequenz ausgewählt aus der Gruppe bestehend aus SEQ ID NOs: 10, 11, 12 und 13.

5. Anti-oxMIF-Antikörper nach einem der Ansprüche 1 bis 2, umfassend die SEQ ID NOs: 6, 11 und eine beliebige der SEQ ID NOs: 1, 2, 3, 4 oder 5.

6. Anti-oxMIF-Antikörper nach einem der Ansprüche 1 bis 2, umfassend die SEQ ID NOs: 7, 10 und eine beliebige der SEQ ID NOs: 1, 2, 3, 4 oder 5.

7. Anti-oxMIF-Antikörper nach einem der Ansprüche 1 bis 2, umfassend die SEQ ID NOs: 7, 11 und eine beliebige der SEQ ID NOs: 1, 2, 3, 4 oder 5.

8. Anti-oxMIF-Antikörper nach einem der Ansprüche 1 bis 2, umfassend die SEQ ID NOs: 8, 11 und eine beliebige der SEQ ID NOs: 1, 2, 3, 4 oder 5.

9. Anti-oxMIF-Antikörper nach einem der Ansprüche 1 bis 2, umfassend die SEQ ID NOs: 8, 13 und eine beliebige der SEQ ID NOs: 1, 2, 3, 4 oder 5.

10. Anti-oxMIF-Antikörper nach einem der Ansprüche 1 bis 2, umfassend die SEQ ID NOs: 8, 10 und eine beliebige der SEQ ID NOs: 1, 2, 3, 4 oder 5.

11. Anti-oxMIF-Antikörper nach einem der Ansprüche 1 bis 3, umfassend SEQ ID NOs: 8, 11, 4 und 38.

12. Anti-oxMIF-Antikörper nach einem der Ansprüche 1 bis 3, umfassend SEQ ID NOs: 8, 13, 4 und 38.

13. Anti-oxMIF-Antikörper nach einem der Ansprüche 1 bis 12, ausgewählt aus der Gruppe bestehend aus bispezifischen Antikörpern, scFv, (scFv)2, scFvFc, Fab, Fab', und F(ab')2, Fab'-SH, Fab-scFv-Fusion, Fab-(scFv)2-Fusion, Fab-scFv-Fc, Fab-(scFv)2-Fc, Fusionsproteinen von zwei Einzelketten-Antikörpern von unterschiedlichen Spezies (BiTE) und Minikörper.

14. Antikörper nach einem der Ansprüche 1 bis 13 zur Verwendung bei der Herstellung eines Medikaments.

15. Pharmazeutische Zusammensetzung, umfassend den Antikörper nach einem der Ansprüche 1 bis 13, gegebenenfalls zusammen mit einem pharmazeutischen Träger oder Adjuvans.

16. Pharmazeutische Zusammensetzung nach Anspruch 15, wobei die Zusammensetzung 10 bis 250 mg/ml des Antikörpers nach einem der Ansprüche 1 bis 13 umfasst, und im Speziellen > 50 mg/ml umfasst.

17. Pharmazeutische Zusammensetzung nach Anspruch 15 oder 16, wobei die pharmazeutische Zusammensetzung für die subkutane Verabreichung formuliert ist.

18. Pharmazeutische Zusammensetzung nach einem der Ansprüche 15 bis 17, wobei die Zusammensetzung für die Verabreichung als die einzige Substanz oder mit einer weiteren pharmazeutischen Zusammensetzung umfassend einen oder mehrere Wirkstoffe, vorzugsweise ausgewählt aus der Gruppe bestehend aus antiviralen, entzündungshemmenden, Antikrebs-, antiangiogenen und antibiotischen Substanzen, vorgesehen ist.

19. Pharmazeutische Zusammensetzung nach einem der Ansprüche 15 bis 18 zur Verwendung bei der Behandlung eines Patienten, der an einer entzündlichen Erkrankung, hyperproliferativen Störung, Infektionserkrankung oder Krebs leidet, im Speziellen bei der Behandlung von Asthma, Vaskulitis, Arthritis, Sepsis, septischem Schock, endotoxischem Schock, toxischem Schocksyndrom, akutem Lungenversagen (ARDS), Glomerulonephritis, entzündlichen Darmerkrankungen, Morbus Crohn, Colitis ulcerosa, Peritonitis, Nephritis und Psoriasis, Darmkrebs, Ovarialkrebs, Brustkrebs, Prostatakrebs, Bauchspeicheldrüsenkrebs und Lungenkrebs.

20. Isolierte Nukleinsäure, die für den Antikörper nach einem der Ansprüche 1 bis 13 kodiert.

21. Expressionsvektor, umfassend das/die Nukleinsäuremolekül(e) nach Anspruch 20.

22. Wirtszelle, die die Nukleinsäure nach Anspruch 20 oder einen Expressionsvektor nach Anspruch 21 enthält.

23. Verfahren zur Produktion des Antikörpers nach einem der Ansprüche 1 bis 13, umfassend das Kultivieren einer Wirtszelle nach Anspruch 22 und das Gewinnen des Antikörpers aus der Zellkultur.

## Revendications

1. Anticorps anti-oxMIF recombinant ou fragment de liaison à l'antigène de celui-ci présentant un potentiel d'agrégation réduit et une hydrophobicité réduite, comprenant les domaines variables suivants :
(a1) un domaine variable de chaîne légère comprenant la SEQ ID N° : 9 avec 1, 2, 3, 4 ou 5 substitutions d'acide aminé choisies parmi M30L, L49Y, A51G, P80S et W93F, ou
(a2) un domaine variable de chaîne légère comprenant la SEQ ID N° : 9 avec 1, 2, 3, 4 ou 5 substitutions d'acide aminé choisies parmi M30L, L49Y, A51G, P80S et W93F, et avec 1, 2, 3, 4 ou 5 substitutions d'acide aminé supplémentaires, à condition que la tyrosine en position 36 soit conservée, et l'un parmi
(b1) un domaine variable de chaîne lourde comprenant la SEQ ID N° : 6 ; ou
(b2) un domaine variable de chaîne lourde comprenant la SEQ ID N° : 6 avec 1 ou 2 substitutions d'acide aminé choisies parmi L5Q et W97Y, ou
(b3) un domaine variable de chaîne lourde comprenant la SEQ ID N° : 6 avec 1 ou 2 substitutions d'acide aminé choisies parmi L5Q et W97Y, et avec 1, 2, 3, 4 ou 5 substitutions d'acide aminé supplémentaires,
dans lequel les positions d'acide aminé sont numérotées conformément à Kabat, et dans lequel le potentiel d'agrégation et l'hydrophobicité sont réduits par rapport à un anticorps ou à un fragment de liaison à l'antigène de celui-ci comprenant la SEQ ID N° 6 et la SEQ ID N° : 9 dépourvues des substitutions d'acide aminé.

2. Anticorps anti-oxMIF recombinant selon la revendication 1, comprenant les substitutions d'acide aminé W93F et/ou W97F.

3. Anticorps anti-oxMIF recombinant selon la revendication 1 ou 2, présentant des fonctions effectrices accrues, comprenant une région constante de chaîne lourde comprenant
(a) la SEQ ID N° : 2 ou une région constante de chaîne lourde présentant une identité de séquence d'au moins 95 % avec la SEQ ID N° : 2 et comprenant les acides aminés 239D et 332F ; ou
(b) la SEQ ID N° : 3 ou une région constante de chaîne lourde présentant une identité de séquence d'au moins 95 % avec la SEQ ID N° : 3 et comprenant les acides aminés 239D, 268F, 324T et 332E ; ou
(c) la SEQ ID N° : 4 ou une région constante de chaîne lourde présentant une identité de séquence d'au moins 95 % avec la SEQ ID N° : 4 et comprenant les acides aminés 239D et 332F ; ou
(d) la SEQ ID N° : 5 ou une région constante de chaîne lourde présentant une identité de séquence d'au moins 95 % avec la SEQ ID N° : 5 et comprenant les acides aminés 235V, 243L, 292P, 300L et 396L,
dans lequel les positions d'acide aminé sont numérotées conformément à un indice de numérotation EU.

4. Anticorps anti-oxMIF selon l'une quelconque des revendications 1 à 3, comprenant la SEQ ID N° : 8 et une séquence d'acides aminés choisie dans le groupe constitué par les SEQ ID N° : 10, 11, 12 et 13.

5. Anticorps anti-oxMIF selon l'une quelconque des revendications 1 à 2, comprenant les SEQ ID N° : 6, 11 et l'une quelconque des SEQ ID N° : 1, 2, 3, 4 ou 5.

6. Anticorps anti-oxMIF selon l'une quelconque des revendications 1 à 2, comprenant les SEQ ID N° : 7, 10 et l'une quelconque des SEQ ID N° : 1, 2, 3, 4 ou 5.

7. Anticorps anti-oxMIF selon l'une quelconque des revendications 1 à 2, comprenant les SEQ ID N° : 7, 11 et l'une quelconque des SEQ ID N° : 1, 2, 3, 4 ou 5.

8. Anticorps anti-oxMIF selon l'une quelconque des revendications 1 à 2, comprenant les SEQ ID N° : 8, 11 et l'une quelconque des SEQ ID N° : 1, 2, 3, 4 ou 5.

9. Anticorps anti-oxMIF selon l'une quelconque des revendications 1 à 2, comprenant les SEQ ID N° : 8, 13 et l'une quelconque des SEQ ID N° : 1, 2, 3, 4 ou 5.

10. Anticorps anti-oxMIF selon l'une quelconque des revendications 1 à 2, comprenant les SEQ ID N° : 8, 10 et l'une quelconque des SEQ ID N° : 1, 2, 3, 4 ou 5.

11. Anticorps anti-oxMIF selon l'une quelconque des revendications 1 à 3, comprenant les SEQ ID N° : 8, 11, 4 et 38.

12. Anticorps anti-oxMIF selon l'une quelconque des revendications 1 à 3, comprenant les SEQ ID N° : 8, 13, 4 et 38.

13. Anticorps anti-oxMIF selon l'une quelconque des revendications 1 à 12, choisi dans le groupe constitué par les anticorps bispécifiques, ScFv, (scFv)2, scFvFc, Fab, Fab' et F(ab')2, Fab'-SH, la fusion Fab-scFv, la fusion Fab-(scFv)2, Fab-scFv-Fc, Fab-(scFv)2-Fc, les protéines de fusion de deux anticorps à chaîne unique d'espèces différentes (BiTE) et un minicorps.

14. Anticorps selon l'une quelconque des
revendications 1 à 13 destiné à être utilisé dans la préparation d'un médicament.

15. Composition pharmaceutique comprenant l'anticorps selon l'une quelconque des revendications 1 à 13, éventuellement conjointement avec un véhicule ou adjuvant pharmaceutique.

16. Composition pharmaceutique selon la revendication 15, ladite composition comprenant 10 à 250 mg/ml de l'anticorps selon l'une quelconque des revendications 1 à 13, comprenant plus particulièrement > 50 mg/ml.

17. Composition pharmaceutique selon la revendication 15 ou 16, ladite composition pharmaceutique étant formulée pour une administration sous-cutanée.

18. Composition pharmaceutique selon l'une quelconque des revendications 15 à 17, la composition étant destinée à être administrée sous la forme d'une substance unique, ou avec une autre composition pharmaceutique comprenant un ou plusieurs principes actifs, de préférence choisis dans le groupe constitué par les substances antivirales, anti-inflammatoires, anticancéreuses, anti-angiogéniques et antibiotiques.

19. Composition pharmaceutique selon l'une quelconque des revendications 15 à 18 destinée à être utilisée dans le traitement d'un patient souffrant d'une maladie inflammatoire, d'un trouble hyperprolifératif, d'une maladie infectieuse ou d'un cancer, plus particulièrement dans le traitement de l'asthme, de la vasculite, de l'arthrite, de la septicémie, d'un choc septique, d'un choc endotoxique, du syndrome de choc toxique, du syndrome de détresse respiratoire acquise, de la glomérulonéphrite, d'une maladie inflammatoire de l'intestin, de la maladie de Crohn, de la colite ulcéreuse, de la péritonite, de la néphrite et du psoriasis, du cancer colorectal, de cancer de l'ovaire, du cancer du sein, du cancer de la prostate, du cancer du pancréas et du cancer du poumon.

20. Acide nucléique isolé codant l'anticorps selon l'une quelconque des revendications 1 à 13.

21. Vecteur d'expression comprenant la ou les molécules d'acide nucléique selon la revendication 20.

22. Cellule hôte contenant l'acide nucléique selon la revendication 20 ou un vecteur d'expression selon la revendication 21.

23. Procédé de production de l'anticorps selon l'une quelconque des revendications 1 à 13, comprenant la culture d'une cellule hôte selon la revendication 22 et la récupération dudit anticorps à partir de la culture de cellules.
